# EUROPEAN PATENT SPECIFICATION

(11) **EP 3 150 204 B1**
(45) Date of publication and mention of the grant of the patent: **09.12.2026**
(21) Application number: 16195824.4
(22) Date of filing: 15.10.2009
(51) Int. Cl.: A61K 31/155, A61K 31/5025, A61K 31/519, A61K 31/522, A61P 3/10

(54) **TREATMENT FOR DIABETES IN PATIENTS WITH INSUFFICIENT GLYCEMIC CONTROL DESPITE THERAPY WITH AN ORAL OR NON-ORAL ANTIDIABETIC DRUG**
DIABETESBEHANDLUNG BEI PATIENTEN MIT UNZUREICHENDER GLYKÄMISCHER KONTROLLE TROTZ THERAPIE MIT ORALEM ODER NICHT-ORALEM ANTIDIABETIKUM
TRAITEMENT DU DIABÈTE CHEZ DES PATIENTS DONT LA RÉGULATION GLYCÉMIQUE EST INSUFFISANTE MALGRÉ UNE THÉRAPIE PAR UN MÉDICAMENT ANTIDIABÉTIQUE ORAL OU NON ORAL

(30) Priority: 16.10.2008 EP 08166829; 16.10.2008 US 105919 P; 05.08.2009 EP 09167304
(43) Date of publication of application: 05.04.2017
(60) Divisional application: 26189855.5 / 4 803 140
(62) Document number(s) of the earlier application(s) in accordance with Art. 76 EPC: 09736932.6 / 2 349 240
(73) Proprietor: Boehringer Ingelheim International GmbH, 55216 Ingelheim am Rhein (DE)
(72) Inventor: Graefe-Mody, Eva Ulrike, 55216 INGELHEIM AM RHEIN (DE); Klein, Thomas, 55216 INGELHEIM AM RHEIN (DE); Mark, Michael, 55216 INGELHEIM AM RHEIN (DE); Woerle, Hans-Juergen, 55216 INGELHEIM AM RHEIN (DE)
(74) Representative: Hoffmann Eitle

(56) References cited:
- EP-A1- 1 671 649
- WO-A1-2004/050658
- WO-A1-2005/085246
- WO-A1-2005/117861
- WO-A1-2007/071738
- WO-A1-2008/017670
- WO-A1-2009/022007
- WO-A1-2009/022008
- WO-A2-2004/018468
- DE-A1- 102004 044 221
- GRAEFE-MODY ET AL.: "The novel DPP-4 inhibitor BI1356...", 2008, XP002561421, Retrieved from the Internet <URL:http://professional.diabetes.org/Content/Posters/2008/p553-P.pdf> [retrieved on 20091221]
- WANG Y ET AL: "BI-1356. Dipeptidyl-peptidase IV inhibitor, antidiabetic agent", DRUGS OF THE FUTURE, PROUS SCIENCE, ES, vol. 33, no. 6, 1 June 2008 (2008-06-01), pages 473 - 477, XP009110227, ISSN: 0377-8282
- PEI Z: "From the bench to the bedside: Dipeptidyl peptidase IV inhibitors, a new class of oral antihyperglycemic agents", CURRENT OPINION IN DRUG DISCOVERY AND DEVELOPMENT, CURRENT DRUGS, LONDON, GB, vol. 11, no. 4, 1 July 2008 (2008-07-01), pages 512 - 532, XP009110228, ISSN: 1367-6733
- ECKHARDT M ET AL: "3,5-Dihydro-imidazo[4,5-d]pyridazin-4-ones: A class of potent DPP-4 inhibitors", BIOORGANIC & MEDICINAL CHEMISTRY LETTERS, PERGAMON, ELSEVIER SCIENCE, GB, vol. 18, no. 11, 1 June 2008 (2008-06-01), pages 3158 - 3162, XP022711188, ISSN: 0960-894X, [retrieved on 20080501]
- W. T. CEFALU: "Animal Models of Type 2 Diabetes: Clinical Presentation and Pathophysiological Relevance to the Human Condition", ILAR JOURNAL, vol. 47, no. 3, 1 January 2006 (2006-01-01), US, pages 186 - 198, XP055503987, ISSN: 1084-2020, DOI: 10.1093/ilar.47.3.186
- ANDREW J. LEWIN: "Efficacy and T olerability of Linagliptin Added to a SulfonylureaRegimen in Patients With Inadequately Controlled Type 2Diabetes Mellitus: An 18-Week, Multicenter, Randomized,Double-Blind, Placebo-Controlled Trial", CLINICAL THERAPEUTICS, vol. 34, no. 9, 1 September 2012 (2012-09-01), pages 1909 - 1919
- SEN SHI: "Linagliptin but not Sitagliptin inhibited transforming growth factor~2-induced endothelial DPP-4 activity and the endothelialmesenchymaltransition", BIOCHEMICAL AND BIOPHYSICAL RESEARCH COMMUNICATIONS, 27 January 2016 (2016-01-27), pages 184 - 190, XP093316357

## Description

The present invention relates to the DPP-4 inhibitor 1-[(4-methyl-quinazolin-2-yl)methyl]-3-methyl-7-(2-butyn-1-yl)-8-(3-(R)-amino-piperidin-1-yl)-xanthine for use in treating and/or preventing metabolic diseases, particularly diabetes (especially type 2 diabetes mellitus) and conditions related thereto, in patients with insufficient glycemic control despite a therapy with a sulphonylurea drug.

Type 2 diabetes mellitus is a common chronic and progressive disease arising from a complex pathophysiology involving the dual endocrine effects of insulin resistance and impaired insulin secretion. The treatment of type 2 diabetes typically begins with diet and exercise, followed by oral antidiabetic monotherapy, and although conventional monotherapy may initially control blood glucose in some patients, it is however associated with a high secondary failure rate. The limitations of single-agent therapy for maintaining glycemic control may be overcome, at least in some patients, and for a limited period of time by combining multiple oral drugs to achieve reductions in blood glucose that cannot be sustained during long-term therapy with single agents. Available data support the conclusion that in most patients with type 2 diabetes monotherapy will fail and treatment with multiple drugs will be required.

But, because type 2 diabetes is a progressive disease, even patients with good initial responses to combination therapy will eventually require an increase of the dosage or further treatment with insulin because the blood glucose level is very difficult to maintain stable for a long period of time. Thus, although existing combination therapy has the potential to enhance glycemic control, it is not without limitations (especially with regard to long term efficacy). Further, many results indicate that the risk for hypoglycemia may increase with traditional combination therapy, and the requirement for multiple medications may also reduce patient compliance. In addition, taking multiple antihyperglycemic drugs increases the potential for pharmacokinetic interactions with other medications that the patient may be taking.

Thus, for many patients, these existing drug therapies result in progressive deterioriation in glycemic control despite treatment and do not sufficiently control glycemia especially over long-term and thus fail to achieve and to maintain metabolic control in advanced or late stage type 2 diabetes, including diabetes with inadequate glycemic control despite conventional oral or non-oral antidiabetic medication, diabetes with secondary drug failure and/or with indication on insulin.

Therefore, although intensive treatment of hyperglycemia can reduce the incidence of chronic damages, many patients with type 2 diabetes remain inadequately treated, partly because of limitations in long term efficacy, tolerability and dosing inconvenience of conventional antihyperglycemic therapies.

This high incidence of therapeutic failure is a major contributor to the high rate of long-term hyperglycemia-associated complications or chronic damages (including micro- and makrovascular complications such as e.g. diabetic nephrophathy, retinopathy or neuropathy, or cardiovascular complications) in patients with type 2 diabetes.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and α-glucosidase inhibitors.

Non-oral antidiabetic drugs conventionally used in therapy (such as e.g. first- or second-line, and/or mono- or (initial or add-on) combination therapy) include, without being restricted thereto, GLP-1 or GLP-1 analogues, and insulin or insulin analogues.

However, the use of these conventional antidiabetic or antihyperglycemic agents can be associated with various adverse effects. For example, metformin can be associated with lactic acidosis or gastrointestinal side effects; sulfonylureas, glinides and insulin or insulin analogues can be associated with hypoglycemia and weight gain; thiazolidinediones can be associated with edema, bone fracture, weight gain and heart failure/cardiac effects; and alpha-glucosidase blockers and GLP-1 or GLP-1 analogues can be associated with gastrointestinal adverse effects (e.g. dyspepsia, flatulence or diarrhea, or nausea or vomiting).

Sulphonylureas (SU), as well as glinides, stimulate insulin secretion from pancreatic betacells in a non-glucose-dependent manner and are generally and frequentially used as a firstor second-line (mono- or combination) treatment in type 2 diabetes (especially indicated for non-obese patients and/or for patients ineligible for or with failure in metformin therapy). However, as mentioned above, some patients do not always respond well to these conventional oral antidiabetic agents especially in long-term treatment and may show insufficient or deterioration in glycemic control despite treatment with a sulphonylurea drug (secondary SU failure). Also, patients on long-term sulfonylurea therapy experience a decline or an exhaustion in pancreatic beta cell function over time.

For those patients who are in a condition in which sulfonylureas alone (monotherapy) are ineffective in controlling blood glucose levels, change to other oral medication (e.g. to metformin/thiazolidinedione) or to SU combination therapy (including add-on and initial dual and triple SU combination therapy), especially combination of a sulfonylurea with metformin and/or with a thiazolidinedione, may be indicated depending on the disease stage, and/or combination with or (ultimately) transfer to insulin (such as e.g., depending on disease stage, once-daily basal insulin, twice-daily premix insulin or multiple daily insulin). However, even in combination therapy, some patients may show insufficient or deterioration in glycemic control despite combination treatment, especially over time.

Thus, continuing loss of efficacy over time is a major concern with the use of insulin secretagogues including glinides and sulfonylureas (secondary SU failure). Furthermore, sulfonylureas increase plasma levels of insulin and may cause hypoglycaemia, which is - besides weight gain - one of their major adverse effects, particularly in association with renal impairment and/or in elderly patients. Thus, within SU medication, on the one side, with regard to efficacy, sometimes an increased sulfonylurea dose may be required, whereas, on the other side, with regard to safety/tolerability, sometimes a decreased sulfonylurea dose may be required, thus requiring often an unsatisfying compromise in SU medication.

Therefore, it remains a need in the art to provide efficacious, safe and tolerable antidiabetic therapies for these patients with advanced or late stage type 2 diabetes mellitus, including patients with inadequate glycemic control on conventional oral and/or non-oral antidiabetic drugs, such as e.g. metformin, sulphonylureas, thiazolidinediones, glinides and/or α-glucosidase inhibitors, and/or GLP-1 or GLP-1 analogues, and/or insulin or insulin analogues.

Further, it remains a need in the art to provide adequate glycemic control for diabetic patients with secondary oral antidiabetic drug failure.

Further, it remains a need in the art to provide prevention (including preventing or slowing the progression) of secondary oral antidiabetic drug failure.

Further, it remains a need in the art to provide prevention or reduction of risk for adverse effects associated with (conventional) antidiabetic therapy.

EP 1 671 649 A1 describes DPP-4 inhibitors as useful for treatment of diabetic patients with SU failure.

Pei, Current Opinion In Drug Discovery & Development, 11(4): 512-532, 2008, reviews DPP-4 inhibitors as new class of oral antihyperglycemic agents including 1-[(4-methyl-quinazolin-2-yl)methyl]-3-methyl-7-(2-butyn-1-yl)-8-(3-(*R*)-amino-piperidin-1-yl)-xanthine (BI1356) and the use of sitagliptin in the treatment of patients with insufficient glycemic control on metformin monotherapy.

Wang et al., Drugs of the Future, 33(6): 473-477, 2008, reviews BI1356 as antidiabetic DPP-4 inhibitor including its use in ongoing clinical trials relating to treatment of patients with insufficient glycemic control.

In the monitoring of the treatment of diabetes mellitus the HbA1c value, the product of a nonenzymatic glycation of the haemoglobin B chain, is of exceptional importance. As its formation depends essentially on the blood sugar level and the life time of the erythrocytes the HbA1c in the sense of a "blood sugar memory" reflects the average blood sugar level of the preceding 4-12 weeks. Diabetic patients whose HbA1c level has been well controlled over a long time by more intensive diabetes treatment (i.e. < 6.5 % of the total haemoglobin in the sample) are significantly better protected from diabetic microangiopathy. The available treatments for diabetes can give the diabetic an average improvement in their HbA1c level of the order of 1.0 - 1.5 %. This reduction in the HbA1C level is not sufficient in all diabetics to bring them into the desired target range of < 7.0 %, preferably < 6.5 % and more preferably < 6 % HbA1c.

Within glycemic control, in addition to improvement of the HbA1c level, other recommended therapeutic goals for type 2 diabetes mellitus patients are improvement of fasting plasma glucose (FPG) and of postprandial plasma glucose (PPG) levels to normal or as near normal as possible. Recommended desired target ranges of preprandial (fasting) plasma glucose are 90-130 mg/dL (or 70-130 mg/dL) or <110 mg/dL, and of two-hour postprandial plasma glucose are <180 mg/dL or <140 mg/dL.

Within the meaning of this invention, patients with insufficient glycemic control despite a therapy with an oral or non-oral antidiabetic drug include, without being limited to, patients having a HbA1c value from 7.0 to 10% (or from 7.5 to 11%, or from 7.5 to 10%) despite treatment with said drug.

An embodiment of diabetic patients with secondary oral antidiabetic drug failure within the meaning of this invention refers to patients ineligible for metformin therapy including
- patients for whom metformin therapy is contraindicated, e.g. patients having one or more contraindications against metformin therapy according to label, such as for example patients with at least one contraindication selected from:
   renal disease, renal impairment or renal dysfunction (e.g., as specified by product information of locally approved metformin),
   dehydration,
   unstable or acute congestive heart failure,
   acute or chronic metabolic acidosis, and
   hereditary galactose intolerance;
   and
- patients who suffer from one or more intolerable side effects attributed to metformin, particularly gastrointestinal side effects associated with metformin, such as for example patients suffering from at least one gastrointestinal side effect selected from:
   nausea,
   vomiting,
   diarrhoea,
   intestinal gas, and
   severe abdominal discomfort.

An embodiment of the patients which may be amenable to the therapies of this invention may include, without being limited, those diabetes patients for whom normal metformin therapy is not appropriate, such as e.g. those diabetes patients who need reduced dose metformin therapy due to reduced tolerability, intolerability or contraindication against metformin or due to (mildly) impaired/reduced renal function (including elderly patients, e.g. ≥ 60-65 years).

Further, due to increased susceptibility for adverse effects, treatment of the elderly patients (≥ 60-70 years) should be often accompanied by careful monitoring of renal function. Metformin is usually not recommended in elderly individuals, particularly ≥ 80 years, unless measurement of creatinine clearance demonstrates that renal function is not reduced. Thus, patients ineligible for metformin therapy may also include, without being limited to, elderly patients, e.g. ≥ 60-65 years or particularly ≥ 80 years.

A further embodiment of diabetic patients with secondary oral antidiabetic drug failure within the meaning of this invention refers to patients having renal disease, renal dysfunction, or insufficiency or impairment of renal function (including mild, moderate and severe renal impairment), e.g. as suggested by elevated serum creatinine levels (e.g. serum creatinine levels above the upper limit of normal for their age, e.g. ≥ 130 - 150 µmol/l, or ≥ 1.5 mg/dl (≥ 136 µmol/l) in men and ≥ 1.4 mg/dl (≥ 124 µmol/l) in women) or abnormal creatinine clearance (e.g. glomerular filtration rate (GFR) ≤ 30 - 60 ml/min).

In this context, for more detailed example, mild renal impairment may be e.g. suggested by a creatinine clearance of 50-80 ml/min (approximately corresponding to serum creatine levels of ≤1.7 mg/dL in men and ≤1.5 mg/dL in women); moderate renal impairment may be e.g. suggested by a creatinine clearance of 30-50 ml/min (approximately corresponding to serum creatine levels of >1.7 to ≤3.0 mg/dL in men and >1.5 to ≤2.5 mg/dL in women); and severe renal impairment may be e.g. suggested by a creatinine clearance of < 30 ml/min (approximately corresponding to serum creatine levels of >3.0 mg/dL in men and >2.5 mg/dL in women). Patients with end-stage renal disease require dialysis.

The enzyme DPP-4 (dipeptidyl peptidase IV) also known as CD26 is a serine protease known to lead to the cleavage of a dipeptide from the N-terminal end of a number of proteins having at their N-terminal end a prolin or alanin residue. Due to this property DPP-4 inhibitors interfere with the plasma level of bioactive peptides including the peptide GLP-1 and are considered to be promising drugs for the treatment of diabetes mellitus.

For example, DPP-4 inhibitors and their uses, particularly their uses in metabolic (especially diabetic) diseases, are disclosed in WO 2002/068420, WO 2004/018467, WO 2004/018468, WO 2004/018469, WO 2004/041820, WO 2004/046148, WO 2005/051950, WO 2005/082906, WO 2005/063750, WO 2005/085246, WO 2006/027204, WO 2006/029769 or WO2007/014886; or in WO 2004/050658, WO 2004/111051, WO 2005/058901 or WO 2005/097798; or in WO 2006/068163, WO 2007/071738 or WO 2008/017670; or in WO 2007/128721 or WO 2007/128761.

As further DPP-4 inhibitors the following compounds can be mentioned:
- Sitagliptin (MK-0431) having the structural formula A below is (3R)-3-amino-1-[3-(trifluoromethyl)-5,6,7,8-tetrahydro-5H-[1,2,4]triazolo[4,3-a]pyrazin-7-yl]-4-(2,4,5-trifluorophenyl)butan-1-one, also named (2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine,

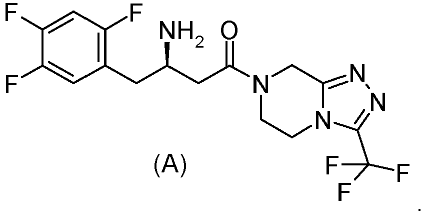

In one embodiment, sitagliptin is in the form of its dihydrogenphosphate salt, i.e. sitagliptin phosphate. In a further embodiment, sitagliptin phosphate is in the form of a crystalline anhydrate or monohydrate. A class of this embodiment refers to sitagliptin phosphate monohydrate. Sitagliptin free base and pharmaceutically acceptable salts thereof are disclosed in US Patent No. 6,699,871 and in Example 7 of WO 03/004498. Crystalline sitagliptin phosphate monohydrate is disclosed in WO 2005/003135 and in WO 2007/050485.

For details, e.g. on a process to manufacture, to formulate or to use this compound or a salt thereof, reference is thus made to these documents.

A tablet formulation for sitagliptin is commercially available under the trade name Januvia^{®}. A tablet formulation for sitagliptin/metformin combination is commercially available under the trade name Janumet^{®}.
- Vildagliptin (LAF-237) having the structural formula B below is (2S)-{[(3-hydroxyadamantan-1-yl)amino]acetyl}pyrrolidine-2-carbonitrile, also named (S)-1-[(3-hydroxy-1-adamantyl)amino]acetyl-2-cyano-pyrrolidine,

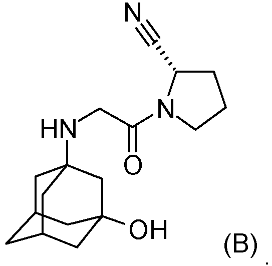

Vildagliptin is specifically disclosed in US Patent No. 6,166,063 and in Example 1 of WO 00/34241. Specific salts of vildagliptin are disclosed in WO 2007/019255. A crystalline form of vildagliptin as well as a vildagliptin tablet formulation are disclosed in WO 2006/078593. Vildagliptin can be formulated as described in WO 00/34241 or in WO 2005/067976. A modified release vildagliptin formulation is described in WO 2006/135723.

For details, e.g. on a process to manufacture, to formulate or to use this compound or a salt thereof, reference is thus made to these documents.

A tablet formulation for vildagliptin is expected to be commercially available under the trade name Galvus^{®}. A tablet formulation for vildagliptin/metformin combination is commercially available under the trade name Eucreas^{®}.
- Saxagliptin (BMS-477118) having the structural formula C below is (1S,3S,5S)-2-{(2S)-2-amino-2-(3-hydroxyadamantan-1-yl)acetyl}-2-azabicyclo[3.1.0]hexane-3-carbonitrile, also named (S)-3-hydroxyadamantylglycine-L-*cis*-4,5-methanoprolinenitrile,

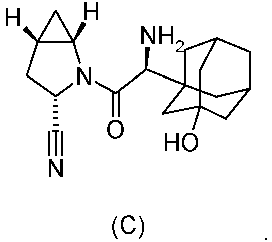

Saxagliptin is specifically disclosed in US Patent No. 6,395,767 and in Example 60 of WO 01/68603.

In one embodiment, saxagliptin is in the form of its HCl salt or its mono-benzoate salt as disclosed in WO 2004/052850. In a further embodiment, saxagliptin is in the form of the free base. In a yet further embodiment, saxagliptin is in the form of the monohydrate of the free base as disclosed in WO 2004/052850. Crystalline forms of the HCl salt and the free base of saxagliptin are disclosed in WO 2008/131149. A process for preparing saxagliptin is also disclosed in WO 2005/106011 and WO 2005/115982. Saxagliptin can be formulated in a tablet as described in WO 2005/117841.

For details, e.g. on a process to manufacture, to formulate or to use this compound or a salt thereof, reference is thus made to these documents.
- Alogliptin (SYR-322) having the structural formula E below is 2-({6-[(3R)-3-aminopiperidin-1-yl]-3-methyl-2,4-dioxo-3,4-dihydro-2H-pyrimidin-1-yl}methyl)benzonitrile

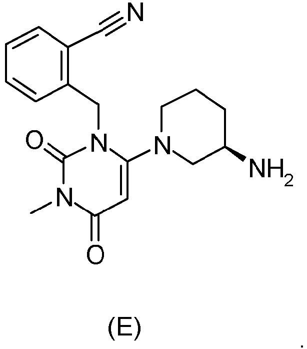

Alogliptin is specifically disclosed in US 2005/261271, EP 1586571 and in WO 2005/095381. In one embodiment, alogliptin is in the form of its benzoate salt, its hydrochloride salt or its tosylate salt each as disclosed in WO 2007/035629. A class of this embodiment refers to alogliptin benzoate. Polymorphs of alogliptin benzoate are disclosed in WO 2007/035372. A process for preparing alogliptin is disclosed in WO 2007/112368 and, specifically, in WO 2007/035629. Alogliptin (namely its benzoate salt) can be formulated in a tablet and administered as described in WO 2007/033266. Formulations of Aloglipitin with metformin or pioglitazone are described in WO 2008/093882 or WO 2009/011451, respectively.

For details, e.g. on a process to manufacture, to formulate or to use this compound or a salt thereof, reference is thus made to these documents.
- (2S)-1-{[2-(5-Methyl-2-phenyl-oxazol-4-yl)-ethylamino]-acetyl}-pyrrolidine-2-carbonitrile or a pharmaceutically acceptable salt thereof, preferably the mesylate, or (2S)-1-{[1,1,-Dimethyl-3-(4-pyridin-3-yl-imidazol-1-yl)-propylamino]-acetyl}-pyrrolidine-2-carbonitrile or a pharmaceutically acceptable salt thereof:

These compounds and methods for their preparation are disclosed in WO 03/037327.

The mesylate salt of the former compound as well as crystalline polymorphs thereof are disclosed in WO 2006/100181. The fumarate salt of the latter compound as well as crystalline polymorphs thereof are disclosed in WO 2007/071576. These compounds can be formulated in a pharmaceutical composition as described in WO 2007/017423.

For details, e.g. on a process to manufacture, to formulate or to use these compounds or salts thereof, reference is thus made to these documents.
- (S)-1-((2S,3S,11bS)-2-Amino-9,10-dimethoxy-1,3,4,6,7,11b-hexahydro-2H-pyrido[2,1-a]isoquinolin-3-yl)-4-fluoromethyl-pyrrolidin-2-one or a pharmaceutically acceptable salt thereof:

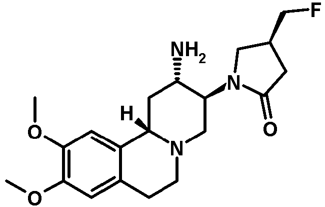

This compound and methods for its preparation are disclosed in WO 2005/000848. A process for preparing this compound (specifically its dihydrochloride salt) is also disclosed in WO 2008/031749, WO 2008/031750 and WO 2008/055814. This compound can be formulated in a pharmaceutical composition as described in WO 2007/017423.

For details, e.g. on a process to manufacture, to formulate or to use this compound or a salt thereof, reference is thus made to these documents.
- (3,3-Difluoropyrrolidin-1-yl)-((2S,4S)-4-(4-(pyrimidin-2-yl)piperazin-1-yl)pyrrolidin-2-yl)methanone (also named gosogliptin) or a pharmaceutically acceptable salt thereof:

This compound and methods for its preparation are disclosed in WO 2005/116014 and US 7291618.

For details, e.g. on a process to manufacture, to formulate or to use this compound or a salt thereof, reference is thus made to these documents.
- (1((3S,4S)-4-amino-1-(4-(3,3-difluoropyrrolidin-1-yl)-1,3,5-triazin-2-yl)pyrrolidin-3-yl)-5,5-difluoropiperidin-2-one or a pharmaceutically acceptable salt thereof:

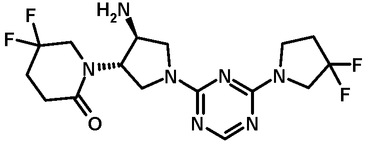

This compound and methods for its preparation are disclosed in WO 2007/148185 and US 20070299076. For details, e.g. on a process to manufacture, to formulate or to use this compound or a salt thereof, reference is thus made to these documents.
- (2S,4S)-1-{2-[(3S,1R)-3-(1H-1,2,4-Triazol-1-ylmethyl)cyclopentylamino]-acetyl}-4-fluoropyrrolidine-2-carbonitrile (also named melogliptin) or a pharmaceutically acceptable salt thereof:

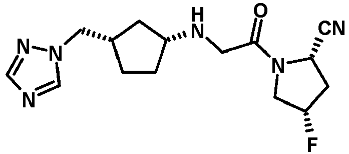

This compound and methods for its preparation are disclosed in WO 2006/040625 and WO 2008/001195. Specifically claimed salts include the methanesulfonate and p-toluenesulfonate. For details, e.g. on a process to manufacture, to formulate or to use this compound or a salt thereof, reference is thus made to these documents.
- (R)-2-[6-(3-Amino-piperidin-1-yl)-3-methyl-2,4-dioxo-3,4-dihydro-2H-pyrimidin-1-ylmethyl]-4-fluoro-benzonitrile or a pharmaceutically acceptable salt thereof:

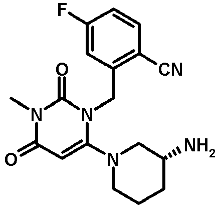

This compound and methods for its preparation and use are disclosed in WO 2005/095381, US 2007060530, WO 2007/033350, WO 2007/035629, WO 2007/074884, WO 2007/112368, WO 2008/114807, WO 2008/114800 and WO 2008/033851. Specifically claimed salts include the succinate (WO 2008/067465), benzoate, benzenesulfonate, p-toluenesulfonate, (R)-mandelate and hydrochloride. For details, e.g. on a process to manufacture, to formulate or to use this compound or a salt thereof, reference is thus made to these documents.
- 5-{(S)-2-[2-((S)-2-Cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide or a pharmaceutically acceptable salt thereof:

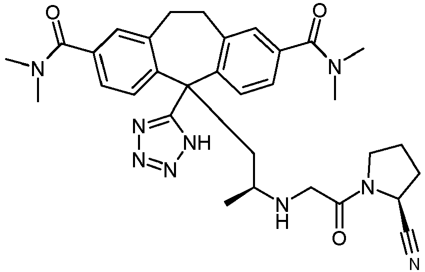

This compound and methods for its preparation are disclosed in WO 2006/116157 and US 2006/270701. For details, e.g. on a process to manufacture, to formulate or to use this compound or a salt thereof, reference is thus made to these documents.
- 3-{(2S,4S)-4-[4-(3-Methyl-1-phenyl-1H-pyrazol-5-yl)piperazin-1-yl]pyrrolidin-2-ylcarbonyl}thiazolidine (also named teneligliptin) or a pharmaceutically acceptable salt thereof:
   This compound and methods for its preparation are disclosed in WO 02/14271. Specific salts are disclosed in WO 2006/088129 and WO 2006/118127 (including hydrochloride, hydrobromide, inter alia). Combination therapy using this compound is described in WO 2006/129785. For details, e.g. on a process to manufacture, to formulate or to use this compound or a salt thereof, reference is thus made to these documents.
- [(2R)-1-{[(3R)-pyrrolidin-3-ylamino]acetyl}pyrrolidin-2-yl]boronic acid (also named dutogliptin) or a pharmaceutically acceptable salt thereof:
   This compound and methods for its preparation are disclosed in WO 2005/047297, WO 2008/109681 and WO 2009/009751. Specific salts are disclosed in WO 2008/027273 (including citrate, tartrate). A formulation of this compound is described in WO 2008/144730. For details, e.g. on a process to manufacture, to formulate or to use this compound or a salt thereof, reference is thus made to these documents.
- (2S,4S)-1-[2-[(4-ethoxycarbonylbicyclo[2.2.2]oct-1-yl)amino]acetyl]-4-fluoropyrrolidine-2-carbonitrile or a pharmaceutically acceptable salt thereof:
   This compound and methods for its preparation are disclosed in WO 2005/075421, US 2008/146818 and WO 2008/114857. For details, e.g. on a process to manufacture, to formulate or to use this compound or a salt thereof, reference is thus made to these documents.
- 2-({6-[(3R)-3-amino-3-methylpiperidin-1-yl]-1,3-dimethyl-2,4-dioxo-1,2,3,4-tetrahydro-5H-pyrrolo[3,2-d]pyrimidin-5-yl}methyl)-4-fluorobenzonitrile or a pharmaceutically acceptable salt thereof, or 6-[(3R)-3-amino-piperidin-1-yl]-5-(2-chloro-5-fluoro-benzyl)-1,3-dimethyl-1,5-dihydro-pyrrolo[3,2-d]pyrimidine-2,4-dione or a pharmaceutically acceptable salt thereof:
   These compounds and methods for their preparation are disclosed in WO 2009/084497 and WO 2006/068163, respectively. For details, e.g. on a process to manufacture, to formulate or to use these compounds or salts thereof, reference is thus made to these documents.

Within the scope of the present invention it has now surprisingly been found that 1-[(4-methyl-quinazolin-2-yl)methyl]-3-methyl-7-(2-butyn-1-yl)-8-(3-(R)-amino-piperidin-1-yl)-xanthine has unexpected and particularly advantageous properties, which makes it particularly suitable for treating and/or preventing (including preventing or slowing the progression or delaying the onset) of metabolic diseases, particularly diabetes (especially type 2 diabetes mellitus) and conditions related thereto (e.g. diabetic complications), in advanced or late stage type 2 diabetes patients, including patients with insufficient glycemic control despite a therapy with an oral and/or a non-oral antidiabetic drug and/or with indication on insulin. The invention is set out in the appended set of claims.

The description may encompass subject-matter extending beyond the scope of the claims. However, the scope of the invention and of protection is defined by the appended claims and shall not extend beyond their scope. In particular, the scope of protection shall not include any method of therapeutic treatment of the human or animal body, even if such subject-matter is disclosed or implied herein.

The present invention provides a DPP-4 inhibitor as defined herein for use in the treatment of patients with insufficient glycemic control despite a therapy (including mono-, dual or triple medication) with one or more conventional oral antidiabetic drugs selected from metformin, sulphonylureas, thiazolidinediones, glinides and α-glucosidase inhibitors.

In another embodiment, the present invention provides a DPP-4 inhibitor as defined herein for use in the treatment of patients with insufficient glycemic control despite therapy (including mono-, dual or triple medication) with one, two or three conventional oral or non-oral antidiabetic drugs selected from metformin, sulphonylureas, thiazolidinediones, glinides, alpha-glucosidase blockers, GLP-1 and GLP-1 analogues, and insulin and insulin analogues; for example, despite mono-therapy with metformin, a sulphonylurea, pioglitazone or (basal) insulin, or despite dual combination therapy with a metformin/pioglitazone, metformin/sulphonylurea, metformin/(basal) insulin, sulphonylurea/pioglitazone, sulphonylurea/(basal) insulin or pioglitazone/(basal) insulin combination.

The present invention further provides a DPP-4 inhibitor as defined herein for use in the treatment of diabetes patients with insufficient glycemic control despite mono-therapy with a sulphonylurea, or despite dual combination therapy with a metformin/sulphonylurea, sulphonylurea/pioglitazone or sulphonylurea/(basal) insulin combination.

In particular, the present invention provides a DPP-4 inhibitor as defined herein for use in the treatment of patients with insufficient glycemic control despite a therapy with a sulphonylurea drug.

The present invention further provides a DPP-4 inhibitor as defined herein for use in the treatment and/or prevention of metabolic diseases, particularly type 2 diabetes mellitus, in patients with insufficient glycemic control despite a therapy with a sulphonylurea drug.

The present invention further provides a DPP-4 inhibitor as defined herein for use in the treatment and/or prevention of diabetes with secondary sulphonylurea failure.

The present invention further provides the use of a DPP-4 inhibitor as defined herein for the manufacture of a pharmaceutical composition for treating and/or preventing metabolic diseases, particularly type 2 diabetes mellitus, in patients with insufficient glycemic control despite a therapy with a sulphonylurea drug.

The present invention further provides a pharmaceutical composition for use in the treatment and/or prevention of metabolic diseases, particularly type 2 diabetes mellitus, in patients with insufficient glycemic control despite a therapy with a sulphonylurea drug, said pharmaceutical composition comprising a DPP-4 inhibitor as defined herein and optionally one or more pharmaceutically acceptable carriers and/or diluents.

The present invention further provides a fixed or non-fixed combination including a kit-of-parts for use in the treatment and/or prevention of metabolic diseases, particularly type 2 diabetes mellitus, in patients with insufficient glycemic control despite a therapy with a sulphonylurea drug, said combination comprising a DPP-4 inhibitor as defined herein and optionally one or more other active substances, e.g. any of those mentioned herein.

The present invention further provides the use of a DPP-4 inhibitor as defined herein in combination with one or more other active substances, such as e.g. any of those mentioned herein, for the manufacture of a pharmaceutical composition for treatment and/or prevention of metabolic diseases, particularly type 2 diabetes mellitus, in patients with insufficient glycemic control despite a therapy with a sulphonylurea drug.

The present invention further provides a pharmaceutical composition for use in the treatment and/or prevention of metabolic diseases, particularly type 2 diabetes mellitus, in patients with insufficient glycemic control despite a therapy with a sulphonylurea drug, said pharmaceutical composition comprising a DPP-4 inhibitor as defined herein and optionally one or more other active substances, such as e.g. any of those mentioned herein, such as e.g. for separate, sequential, simultaneous, concurrent or chronologically staggered use of the active ingredients.

The present invention further provides a method of treating and/or preventing metabolic diseases, particularly type 2 diabetes mellitus, in patients with insufficient glycemic control despite a therapy with a sulphonylurea drug, said method comprising administering to a subject in need thereof (particularly a human patient) an effective amount of a DPP-4 inhibitor as defined herein, optionally alone or in combination, such as e.g. separately, sequentially, simultaneously, concurrently or chronologically staggered, with an effective amount of one or more other active substances, such as e.g. any of those mentioned herein.

In addition, the present invention provides a DPP-4 inhibitor as defined herein, optionally in (add-on or initial) combination with one or two conventional antihyperglycemic agents selected from metformin, sulphonylureas, thiazolidinediones (e.g. pioglitazone), glinides, alpha-glucosidase blockers, GLP-1 or GLP-1 analogues, and insulin or insulin analogues, for use in patients with insufficient glycemic control despite therapy with (e.g., if applicable, despite therapy with a maximal tolerated oral dose of) one, two or three conventional antihyperglycemic agents selected from metformin, sulphonylureas, thiazolidinediones, glinides, alpha-glucosidase blockers, GLP-1 or GLP-1 analogues, and insulin or insulin analogues (e.g. despite mono-therapy with metformin, a sulphonylurea, pioglitazone or (basal) insulin, or despite dual combination therapy with a metformin/pioglitazone, metformin/sulphonylurea, metformin/(basal) insulin, sulphonylurea/pioglitazone, sulphonylurea/(basal) insulin or pioglitazone/(basal) insulin combination).

In a further embodiment of the present invention, it is provided a DPP-4 inhibitor as defined herein, optionally in combination with one conventional antihyperglycemic agent selected from metformin, sulphonylureas, thiazolidinediones (e.g. pioglitazone), glinides, alpha-glucosidase blockers, GLP-1 and GLP-1 analogues, and insulin and insulin analogues, for use in (second line) therapy of type 2 diabetes patients who are insufficiently controlled on said conventional antihyperglycemic agent alone.

In a further embodiment of the present invention, it is provided a DPP-4 inhibitor as defined herein, optionally in combination with two conventional antihyperglycemic agents selected from metformin, sulphonylureas, thiazolidinediones (e.g. pioglitazone), glinides, alpha-glucosidase blockers, GLP-1 and GLP-1 analogues, and insulin and insulin analogues, for use in (third line) therapy of type 2 diabetes patients who are insufficiently controlled on a dual combination of said conventional antihyperglycemic agents.

In a further embodiment of the present invention, it is provided a DPP-4 inhibitor as defined herein, in combination with a conventional antihyperglycemic agent selected from the group consisting of metformin, pioglitazone, a sulphonylurea, and insulin; for use in therapy of type 2 diabetes patients with insufficient glycemic control on the conventional antihyperglycemic agent alone.

In a further embodiment of the present invention, it is provided a DPP-4 inhibitor as defined herein, in combination with two conventional antihyperglycemic agents selected from the group consisting of the following combinations: metformin and pioglitazone, metformin and a sulphonylurea, metformin and insulin, a sulphonylurea and pioglitazone, a sulphonylurea and insulin, and pioglitazone and insulin; for use in therapy of type 2 diabetes patients with insufficient glycemic control on the two conventional antihyperglycemic agents.

In particular, the present invention provides a DPP-4 inhibitor as defined herein in combination with a sulphonylurea for use in the treatment of type 2 diabetes patients with insufficient glycemic control despite mono-therapy with a maximal tolerated dose of a sulphonylurea.

Further, the present invention provides a DPP-4 inhibitor as defined herein in combination with a sulphonylurea and metformin for use in the treatment of type 2 diabetes patients with insufficient glycemic control despite dual combination therapy with a sulphonylurea and metformin.

Further, the present invention provides a DPP-4 inhibitor as defined herein in combination with a sulphonylurea and pioglitazone for use in the treatment of type 2 diabetes patients with insufficient glycemic control despite dual combination therapy with a sulphonylurea and pioglitazone.

Further, the present invention provides a DPP-4 inhibitor as defined herein in combination with a sulphonylurea and insulin for use in the treatment of type 2 diabetes patients with insufficient glycemic control despite dual combination therapy with a sulphonylurea and insulin.

Further, the DPP-4 inhibitors as defined herein may be useful in one or more of the following methods
- for preventing, slowing progression of, delaying, or treating a metabolic disorder;
- for improving glycemic control and/or for reducing of fasting plasma glucose, of postprandial plasma glucose and/or of glycosylated hemoglobin HbA1c;
- for preventing, slowing progression of, delaying or treating of a condition or disorder selected from the group consisting of complications of diabetes mellitus;
- for reducing the weight or preventing an increase of the weight or facilitating a reduction of the weight;
- for preventing or treating the degeneration of pancreatic beta cells and/or for improving and/or restoring the functionality of pancreatic beta cells and/or stimulating and/or restoring the functionality of pancreatic insulin secretion; and/or
- for maintaining and/or improving the insulin sensitivity and/or for treating or preventing hyperinsulinemia and/or insulin resistance;
in diabetes patients with insufficient glycemic control despite a therapy with an oral antidiabetic drug, particularly a sulphonylurea drug (secondary SU failure).

Examples of such metabolic diseases or disorders amenable by the therapy of this invention in patients with secondary oral antidiabetic drug failure may include, without being restricted to, Type 1 diabetes, Type 2 diabetes, inadequate glucose tolerance, insulin resistance, hyperglycemia, hyperlipidemia, hypercholesterolemia, dyslipidemia, metabolic syndrome X, obesity, hypertension, chronic systemic inflammation, retinopathy, neuropathy, nephropathy, atherosclerosis, endothelial dysfunction and osteoporosis.

The present invention further provides the use of a DPP-4 inhibitor as defined herein, optionally in combination with one or more other active substances, such as e.g. any of those mentioned herein, for the manufacture of a medicament for one or more of the following purposes:
- preventing, slowing the progression of, delaying or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, non alcoholic fatty liver disease (NAFLD), retinopathy, neuropathy, nephropathy and/or metabolic syndrome;
- improving glycemic control and/or for reducing of fasting plasma glucose, of postprandial plasma glucose and/or of glycosylated hemoglobin HbA1c;
- preventing, slowing, delaying or reversing progression from impaired glucose tolerance (IGT), impaired fasting blood glucose (IFG), insulin resistance and/or from metabolic syndrome to type 2 diabetes mellitus;
- preventing, reducing the risk of, slowing the progression of, delaying or treating of complications of diabetes mellitus such as micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight or preventing an increase in body weight or facilitating a reduction in body weight;
- preventing, slowing, delaying or treating the degeneration of pancreatic beta cells and/or the decline of the functionality of pancreatic beta cells and/or for improving and/or restoring the functionality of pancreatic beta cells and/or stimulating and/or restoring the functionality of pancreatic insulin secretion;
- preventing, slowing, delaying or treating non alcoholic fatty liver disease (NAFLD) including hepatic steatosis, non-alcoholic steatohepatitis (NASH) and/or liver fibrosis;
- preventing, slowing the progression of, delaying or treating type 2 diabetes with primary or secondary failure to conventional (oral) antihyperglycemic mono- or combination therapy;
- achieving a reduction in the dose of conventional antihyperglycemic medication required for adequate therapeutic effect;
- reducing the risk for adverse effects associated with conventional antihyperglycemic medication; and/or
- maintaining and/or improving the insulin sensitivity and/or for treating or preventing hyperinsulinemia and/or insulin resistance;
particularly in a patient with insufficient glycemic control despite mono- or dual or triple combination therapy with conventional oral or non-oral antidiabetic drug(s) selected from metformin, sulphonylureas, thiazolidinediones (e.g. pioglitazone), glinides, alpha-glucosidase blockers, GLP-1 and GLP-1 analogues, and insulin and insulin analogues.

A special embodiment of this invention refers to a DPP-4 inhibitor as defined herein for use in attaining and/or maintaining glycemic control in type 2 diabetes patients with secondary sulphonylurea failure.

Another special embodiment of this invention refers to a DPP-4 inhibitor as defined herein for use in preventing (including preventing or slowing the progession) of diabetes with secondary SU failure.

Another special embodiment of this invention refers to a DPP-4 inhibitor as defined herein for use in preventing or reducing the risk for adverse effects associated with SU antidiabetic therapy, such as e.g. hypoglycaemia and/or weight gain (or even for use in obtaining weight loss).

Another special embodiment of this invention refers to a DPP-4 inhibitor as defined herein for use in the treatment of diabetes with secondary sulphonylurea failure, wherein said DPP-4 inhibitor is used in add-on or initial combination therapy with a sulphonylurea mono- or dual medication (e.g. as add-on therapy to a SU medication with or without metformin) or as replacement of a sulphonylurea medication, optionally in combination with one or more other therapeutic agents, such as e.g. metformin and/or thiazolidinedione (e.g. pioglitazone).

Another special embodiment of this invention refers to a DPP-4 inhibitor as defined herein for use in improving (e.g. mean reduction from baseline) HbA1c and/or FPG, decreasing glucose excursion and/or improving insulin secretion in patients with insufficient glycemic control (e.g. having HbA1c from 7.5 to 10% or from 7.5 to 11%) despite a therapy with a sulphonylurea drug (such as e.g. glibenclamide, glipizide or glimepiride, with or without metformin).

Other aspects of the present invention become apparent to the skilled person from the foregoing and following remarks.

A DPP-4 inhibitor within the meaning of the present invention includes, without being limited to, any of those DPP-4 inhibitors mentioned hereinabove and hereinbelow, preferably orally active DPP-4 inhibitors.

An embodiment of this invention refers to a DPP-4 inhibitor for use in the treatment and/or prevention of metabolic diseases (particularly type 2 diabetes mellitus) in type 2 diabetes patients with secondary oral antidiabetic drug failure, wherein said patients further suffering from renal disease, renal dysfunction or renal impairment, particularly characterized in that said DPP-4 inhibitor is administered to said patients in the same dose levels as to patients with normal renal function, thus e.g. said DPP-4 inhibitor does not require downward dosing adjustment for impaired renal function.

Another embodiment of this invention refers to a DPP-4 inhibitor for use in the treatment and/or prevention of metabolic diseases (particularly type 2 diabetes mellitus) in type 2 diabetes patients with secondary oral antidiabetic drug failure, wherein said patients are also with failure in or ineligible for metformin therapy or in need of metformin dose reduction due to intolerability or contraindication against metformin, such as e.g. any of those intolerabilities or contraindications defined hereinbefore or hereinafter.

A DPP-4 inhibitor which may be suggested for the aforementioned purpose of the present invention (especially for patients with impaired renal function) may be such an oral DPP-4 inhibitor, which and whose active metabolites have preferably a relatively wide (e.g. about > 100 fold) therapeutic window and/or, especially, that are primarily eliminated via hepatic metabolism or biliary excretion.

In more detail, a DPP-4 inhibitor particularly suitable for the aforementioned purpose of the present invention (especially for patients with impaired renal function) may be such an orally administered DPP-4 inhibitor, which has a relatively wide (e.g. > 100 fold) therapeutic window and/or which fulfils one or more of the following pharmacokinetic properties (preferably at its therapeutic oral dose levels):
- The DPP-4 inhibitor is substantially or mainly excreted via the liver (e.g. > 80 % or even > 90 % of the administered oral dose), and/or for which renal excretion represents no substantial or only a minor elimination pathway (e.g. < 10 %, preferably < 7 %, of the administered oral dose measured, for example, by following elimination of a radiolabelled carbon (¹⁴C) substance oral dose);
- The DPP-4 inhibitor is excreted mainly unchanged as parent drug (e.g. with a mean of > 70%, or > 80%, or, preferably, 90% of excreted radioactivity in urine and faeces after oral dosing of radiolabelled carbon (¹⁴C) substance), and/or which is eliminated to a non-substantial or only to a minor extent via metabolism (e.g. < 30%, or < 20%, or, preferably, 10%);
- The (main) metabolite(s) of the DPP-4 inhibitor is/are pharmacologically inactive. Such as e.g. the main metabolite does not bind to the target enzyme DPP-4 and, optionally, it is rapidly eliminated compared to the parent compound (e.g. with a terminal half-life of the metabolite of ≤ 20 h, or, preferably, ≤ about 16 h, such as e.g. 15.9 h).

Further properties of the DPP-4 inhibitor, which may be attractive for the aforementioned purpose of the present invention, may be one or more of the following: Rapid attainment of steady state (e.g. reaching steady state plasma levels (> 90% of the steady state plasma concentration) between second and fifth day of treatment with therapeutic oral dose levels), little accumulation (e.g. with a mean accumulation ratio R_{A,AUC} ≤ 1.4 with therapeutic oral dose levels), and/or preserving a long-lasting effect on DPP-4 inhibition, preferably when used once-daily (e.g. with almost complete (> 90%) DPP-4 inhibition at therapeutic oral dose levels, > 80% inhibition over a 24h interval after once-daily intake of therapeutic oral drug dose), significant decrease in 2h postprandial blood glucose excursions by ≥ 80 % (already on first day of therapy) at therapeutic dose levels, and cumulative amount of unchanged parent compound excreted in urine on first day being below 1% of the administered dose and increasing to not more than about 3-6% in steady state.

Thus, this invention refers also to a DPP-4 inhibitor for use in the treatment and/or prevention of metabolic diseases (in particular type 2 diabetes mellitus in patients for whom metformin therapy is inappropriate due to intolerability or contraindication against metformin, in more particular in patients with renal disease, renal dysfunction or renal impairment), characterized in that said DPP-4 inhibitor is excreted to a non-substantial or only to a minor extent (e.g. < 10 %, preferably < 7 % of administered oral dose) via the kidney (measured, for example, by following elimination of a radiolabelled carbon (¹⁴C) substance oral dose).

Further, this invention refers also to a DPP-4 inhibitor for use in the treatment and/or prevention of metabolic diseases (in particular type 2 diabetes mellitus in patients for whom metformin therapy is inappropriate due to intolerability or contraindication against metformin, in more particular in patients with renal disease, renal dysfunction or renal impairment), characterized in that said DPP-4 inhibitor is excreted substantially or mainly via the liver (measured, for example, by following elimination of a radiolabelled carbon (¹⁴C) substance oral dose).

Further, this invention refers also to a DPP-4 inhibitor for use in the treatment and/or prevention of metabolic diseases (in particular type 2 diabetes mellitus in patients for whom metformin therapy is inappropriate due to intolerability or contraindication against metformin, in more particular in patients with renal disease, renal dysfunction or renal impairment),
characterized in that
said DPP-4 inhibitor is excreted mainly unchanged as parent drug (e.g. with a mean of > 70%, or > 80%, or, preferably, 90 % of excreted radioactivity in urine and faeces after oral dosing of radiolabelled carbon (¹⁴C) substance),
said DPP-4 inhibitor is eliminated to a non-substantial or only to a minor extent via metabolism, and/or
the main metabolite of said DPP-4 inhibitor is pharmacologically inactive or has a relatively wide therapeutic window.

In a first embodiment (embodiment **A**), a DPP-4 inhibitor in the context of the present invention is any DPP-4 inhibitor of
formula (I) 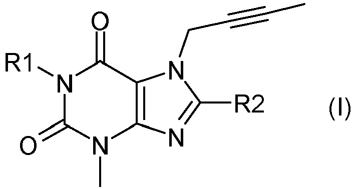 or formula (II) 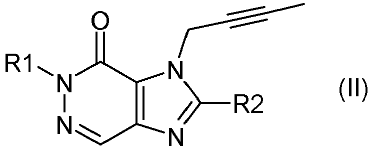 or formula (III) 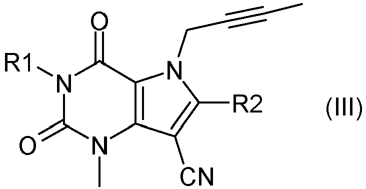 or formula (IV) 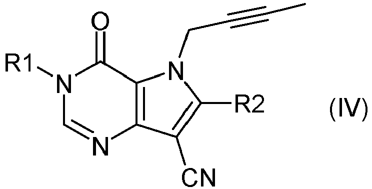
wherein **R1** denotes ([1,5]naphthyridin-2-yl)methyl, (quinazolin-2-yl)methyl, (quinoxalin-6-yl)methyl, (4-methyl-quinazolin-2-yl)methyl, 2-cyano-benzyl, (3-cyano-quinolin-2-yl)methyl, (3-cyano-pyridin-2-yl)methyl, (4-methyl-pyrimidin-2-yl)methyl, or (4,6-dimethyl-pyrimidin-2-yl)methyl and **R2** denotes 3-(R)-amino-piperidin-1-yl, (2-amino-2-methyl-propyl)-methylamino or (2-(S)-amino-propyl)-methylamino,
or its pharmaceutically acceptable salt.

In a second embodiment (embodiment **B**), a DPP-4 inhibitor in the context of the present invention is a DPP-4 inhibitor selected from the group consisting of
sitagliptin, vildagliptin, saxagliptin, alogliptin,
(2S)-1-{[2-(5-Methyl-2-phenyl-oxazol-4-yl)-ethylamino]-acetyl}-pyrrolidine-2-carbonitrile,
(2S)-1-{[1,1,-Dimethyl-3-(4-pyridin-3-yl-imidazol-1-yl)-propylamino]-acetyl}-pyrrolidine-2-carbonitrile,
(S)-1-((2S,3S,11bS)-2-Amino-9,10-dimethoxy-1,3,4,6,7,11b-hexahydro-2H-pyrido[2,1-a]isoquinolin-3-yl)-4-fluoromethyl-pyrrolidin-2-one,
(3,3-Difluoropyrrolidin-1-yl)-((2S,4S)-4-(4-(pyrimidin-2-yl)piperazin-1-yl)pyrrolidin-2-yl)methanone,
(1((3S,4S)-4-amino-1-(4-(3,3-difluoropyrrolidin-1-yl)-1,3,5-triazin-2-yl)pyrrolidin-3-yl)-5,5-difluoropiperidin-2-one,
(2S,4S)-1-{2-[(3S,1R)-3-(1H-1,2,4-Triazol-1-ylmethyl)cyclopentylamino]-acetyl}-4-fluoropyrrolidine-2-carbonitrile,
(R)-2-[6-(3-Amino-piperidin-1-yl)-3-methyl-2,4-dioxo-3,4-dihydro-2H-pyrimidin-1-ylmethyl]-4-fluoro-benzonitrile,
5-{(S)-2-[2-((S)-2-Cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide,
3-{(2S,4S)-4-[4-(3-Methyl-1-phenyl-1H-pyrazol-5-yl)piperazin-1-yl]pyrrolidin-2-ylcarbonyl}thiazolidine,
[(2R)-1-{[(3R)-pyrrolidin-3-ylamino]acetyl}pyrrolidin-2-yl]boronic acid,
(2S,4S)-1-[2-[(4-ethoxycarbonylbicyclo[2.2.2]oct-1-yl)amino]acetyl]-4-fluoropyrrolidine-2-carbonitrile,
2-({6-[(3R)-3-amino-3-methylpiperidin-1-yl]-1,3-dimethyl-2,4-dioxo-1,2,3,4-tetrahydro-5H-pyrrolo[3,2-d]pyrimidin-5-yl}methyl)-4-fluorobenzonitrile, and
6-[(3R)-3-amino-piperidin-1-yl]-5-(2-chloro-5-fluoro-benzyl)-1,3-dimethyl-1,5-dihydro-pyrrolo[3,2-d]pyrimidine-2,4-dione,
or its pharmaceutically acceptable salt.

Regarding the first embodiment (embodiment A), preferred DPP-4 inhibitors are any or all of the following compounds and their pharmaceutically acceptable salts:
- 1-[(4-methyl-quinazolin-2-yl)methyl]-3-methyl-7-(2-butyn-1-yl)-8-(3-(R)-amino-piperidin-1-yl)-xanthine (compare WO 2004/018468, example 2(142)): 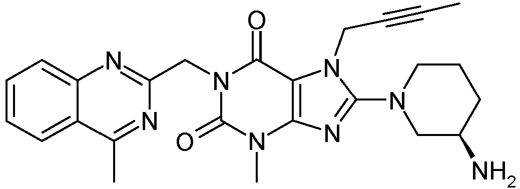
- 1-[([1,5]naphthyridin-2-yl)methyl]-3-methyl-7-(2-butyn-1-yl)-8-((R)-3-amino-piperidin-1-yl)-xanthine (compare WO 2004/018468, example 2(252)): 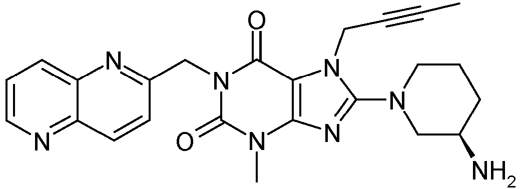
- 1-[(Quinazolin-2-yl)methyl]-3-methyl-7-(2-butyn-1-yl)-8-((R)-3-amino-piperidin-1-yl)-xanthine (compare WO 2004/018468, example 2(80)): 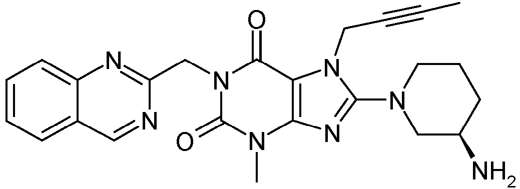
- 2-((R)-3-Amino-piperidin-1-yl)-3-(but-2-yinyl)-5-(4-methyl-quinazolin-2-ylmethyl)-3,5-dihydro-imidazo[4,5-d]pyridazin-4-one (compare WO 2004/050658, example 136): 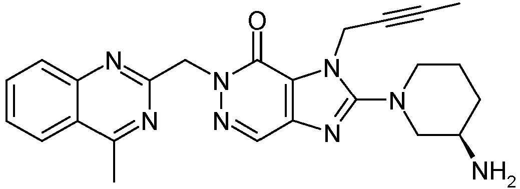
- 1-[(4-Methyl-quinazolin-2-yl)methyl]-3-methyl-7-(2-butyin-1-yl)-8-[(2-amino-2-methylpropyl)-methylamino]-xanthine (compare WO 2006/029769, example 2(1)): 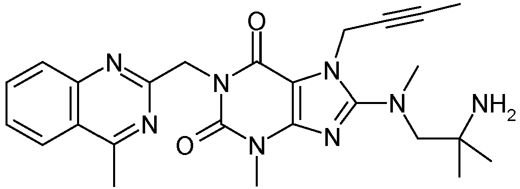
- 1-[(3-Cyano-quinolin-2-yl)methyl]-3-methyl-7-(2-butyn-1-yl)-8-((R)-3-amino-piperidin-1-yl)-xanthine (compare WO 2005/085246, example 1(30)): 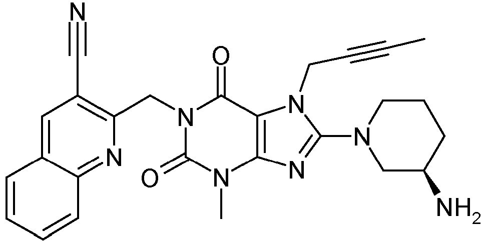
- 1-(2-Cyano-benzyl)-3-methyl-7-(2-butyn-1-yl)-8-((*R*)-3-amino-piperidin-1-yl)-xanthine (compare WO 2005/085246, example 1(39)): 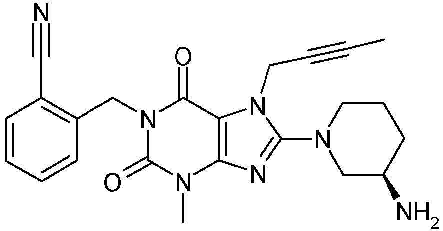
- 1-[(4-Methyl-quinazolin-2-yl)methyl]-3-methyl-7-(2-butyn-1-yl)-8-[(S)-(2-amino-propyl)-methylamino]-xanthine (compare WO 2006/029769, example 2(4)): 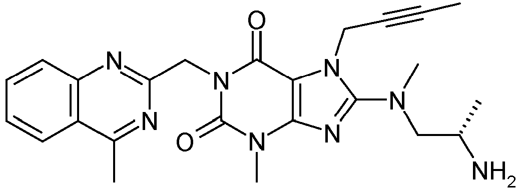
- 1-[(3-Cyano-pyridin-2-yl)methyl]-3-methyl-7-(2-butyn-1-yl)-8-((*R*)-3-amino-piperidin-1-yl)-xanthine (compare WO 2005/085246, example 1(52)): 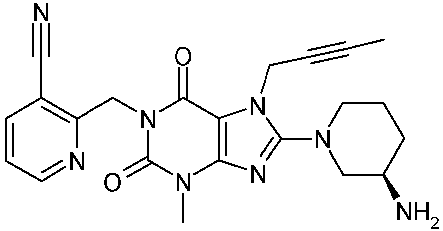
- 1-[(4-Methyl-pyrimidin-2-yl)methyl]-3-methyl-7-(2-butyn-1-yl)-8-((R)-3-amino-piperidin-1-yl)-xanthine (compare WO 2005/085246, example 1(81)): 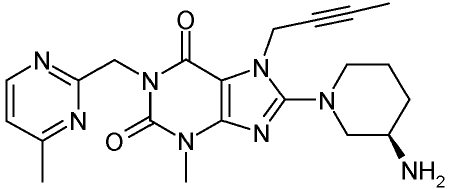
- 1-[(4,6-Dimethyl-pyrimidin-2-yl)methyl]-3-methyl-7-(2-butyn-1-yl)-8-((*R*)-3-amino-piperidin-1-yl)-xanthine (compare WO 2005/085246, example 1(82)): 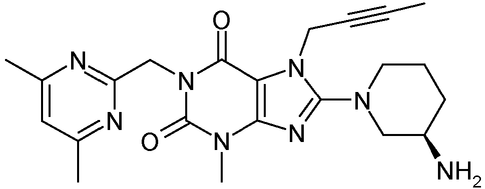
- 1-[(Quinoxalin-6-yl)methyl]-3-methyl-7-(2-butyn-1-yl)-8-((*R*)-3-amino-piperidin-1-yl)-xanthine (compare WO 2005/085246, example 1(83)):

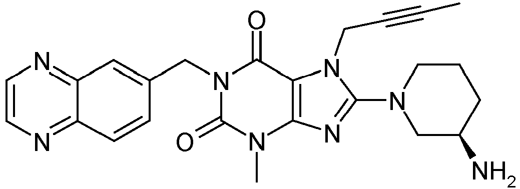

These DPP-4 inhibitors are distinguished from structurally comparable DPP-4 inhibitors, as they combine exceptional potency and a long-lasting effect with favourable pharmacological properties, receptor selectivity and a favourable side-effect profile or bring about unexpected therapeutic advantages or improvements when combined with other pharmaceutical active substances. Their preparation is disclosed in the publications mentioned.

A more preferred DPP-4 inhibitor among the abovementioned DPP-4 inhibitors of embodiment **A** of this invention is 1-[(4-methyl-quinazolin-2-yl)methyl]-3-methyl-7-(2-butyn-1-yl)-8-(3-(*R*)-amino-piperidin-1-yl)-xanthine, particularly the free base thereof (which is also known as BI 1356).

Unless otherwise noted, according to this invention it is to be understood that the definitions of the active compounds (including the DPP-4 inhibitors) mentioned hereinabove and hereinbelow also comprise their pharmaceutically acceptable salts as well as hydrates, solvates and polymorphic forms thereof. With respect to salts, hydrates and polymorphic forms thereof, particular reference is made to those which are referred to herein.

With respect to embodiment **A,** the methods of synthesis for the DPP-4 inhibitors according to embodiment **A** of this invention are known to the skilled person. Advantageously, the DPP-4 inhibitors according to embodiment **A** of this invention can be prepared using synthetic methods as described in the literature. Thus, for example, purine derivatives of formula (I) can be obtained as described in WO 2002/068420, WO 2004/018468, WO 2005/085246, WO 2006/029769 or WO 2006/048427. Purine derivatives of formula (II) can be obtained as described, for example, in WO 2004/050658 or WO 2005/110999. Purine derivatives of formula (III) and (IV) can be obtained as described, for example, in WO 2006/068163, WO 2007/071738 or WO 2008/017670. The preparation of those DPP-4 inhibitors, which are specifically mentioned hereinabove, is disclosed in the publications mentioned in connection therewith. Polymorphous crystal modifications and formulations of particular DPP-4 inhibitors are disclosed in WO 2007/128721 and WO 2007/128724, respectively. Formulations of particular DPP-4 inhibitors with metformin or other combination partners are described in PCT/EP2009053978. Typical dosage strengths of the dual combination of BI 1356 / metformin are 2.5/500 mg, 2.5/850 mg and 2.5/1000 mg.

With respect to embodiment **B,** the methods of synthesis for the DPP-4 inhibitors of embodiment **B** are described in the scientific literature and/ or in published patent documents, particularly in those cited herein.

For pharmaceutical application in warm-blooded vertebrates, particularly humans, the compounds of this invention are usually used in dosages from 0.001 to 100 mg/kg body weight, preferably at 0.1-15 mg/kg, in each case 1 to 4 times a day. For this purpose, the compounds, optionally combined with other active substances, may be incorporated together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof into conventional galenic preparations such as plain or coated tablets, capsules, powders, suspensions or suppositories.

The pharmaceutical compositions according to this invention comprising the DPP-4 inhibitors as defined herein are thus prepared by the skilled person using pharmaceutically acceptable formulation excipients as described in the art. Examples of such excipients include, without being restricted to diluents, binders, carriers, fillers, lubricants, flow promoters, crystallisation retardants, disintegrants, solubilizers, colorants, pH regulators, surfactants and emulsifiers.

Examples of suitable diluents for compounds according to embodiment **A** include cellulose powder, calcium hydrogen phosphate, erythritol, low substituted hydroxypropyl cellulose, mannitol, pregelatinized starch or xylitol. Among those diluents mannitol, low substituted hydroxypropyl cellulose and pregelatinized starch are to be emphasized.

Examples of suitable lubricants for compounds according to embodiment **A** include talc, polyethyleneglycol, calcium behenate, calcium stearate, hydrogenated castor oil or magnesium stearate. Among those lubricants magnesium stearate is to be emphasized.

Examples of suitable binders for compounds according to embodiment **A** include copovidone (copolymerisates of vinylpyrrolidon with other vinylderivates), hydroxypropyl methylcellulose (HPMC), hydroxypropylcellulose (HPC), polyvinylpyrrolidon (povidone), pregelatinized starch, or low-substituted hydroxypropylcellulose (L-HPC). Among those binders copovidone and pregelatinized starch are to be emphasized.

Examples of suitable disintegrants for compounds according to embodiment **A** include corn starch or crospovidone. Among those disintegrants corn starch is to be emphasized.

Suitable methods of preparing pharmaceutical formulations of the DPP-4 inhibitors according to embodiment **A** of the invention are
- direct tabletting of the active substance in powder mixtures with suitable tabletting excipients;
- granulation with suitable excipients and subsequent mixing with suitable excipients and subsequent tabletting as well as film coating; or
- packing of powder mixtures or granules into capsules.

Suitable granulation methods are
- wet granulation in the intensive mixer followed by fluidised bed drying;
- one-pot granulation;
- fluidised bed granulation; or
- dry granulation (e.g. by roller compaction) with suitable excipients and subsequent tabletting or packing into capsules.

An exemplary composition of a DPP-4 inhibitor according to embodiment **A** of the invention comprises the first diluent mannitol, pregelatinized starch as a second diluent with additional binder properties, the binder copovidone, the disintegrant corn starch, and magnesium stearate as lubricant; wherein copovidone and/or corn starch may be optional.

For details on dosage forms, formulations and administration of DPP-4 inhibitors of this invention, reference is made to scientific literature and/ or published patent documents, particularly to those cited herein.

With respect to the first embodiment (embodiment **A**), the dosage typically required of the DPP-4 inhibitors mentioned herein in embodiment **A** when administered intravenously is 0.1 mg to 10 mg, preferably 0.25 mg to 5 mg, and when administered orally is 0.5 mg to 100 mg, preferably 2.5 mg to 50 mg or 0.5 mg to 10 mg, more preferably 2.5 mg to 10 mg or 1 mg to 5 mg, in each case 1 to 4 times a day. Thus, e.g. the dosage of 1-[(4-methylquinazolin-2-yl)methyl]-3-methyl-7-(2-butyn-1-yl)-8-(3-(R)-amino-piperidin-1-yl)-xanthine when administered orally is 0.5 mg to 10 mg per patient per day, preferably 2.5 mg to 10 mg or 1 mg to 5 mg per patient per day.

A dosage form prepared with a pharmaceutical composition comprising a DPP-4 inhibitor mentioned herein in embodiment **A** contain the active ingredient in a dosage range of 0.1-100 mg. Thus, e.g. particular dosage strengths of 1-[(4-methyl-quinazolin-2-yl)methyl]-3-methyl-7-(2-butyn-1-yl)-8-(3-(R)-amino-piperidin-1-yl)-xanthine are 0.5 mg, 1 mg, 2.5 mg, 5 mg and 10 mg.

With respect to the second embodiment (embodiment **B**), the doses of DPP-4 inhibitors mentioned herein in embodiment **B** to be administered to mammals, for example human beings, of, for example, approximately 70 kg body weight, may be generally from about 0.5 mg to about 350 mg, for example from about 10 mg to about 250 mg, preferably 20-200 mg, more preferably 20-100 mg, of the active moiety per person per day, or from about 0.5 mg to about 20 mg, preferably 2.5-10 mg, per person per day, divided preferably into 1 to 4 single doses which may, for example, be of the same size. Single dosage strengths comprise, for example, 10, 25, 40, 50, 75, 100, 150 and 200 mg of the DPP-4 inhibitor active moiety.

A dosage strength of the DPP-4 inhibitor sitagliptin is usually between 25 and 200 mg of the active moiety. A recommended dose of sitagliptin is 100 mg calculated for the active moiety (free base anhydrate) once daily. Unit dosage strengths of sitagliptin free base anhydrate (active moiety) are 25, 50, 75, 100, 150 and 200 mg. Particular unit dosage strengths of sitagliptin (e.g. per tablet) are 25, 50 and 100 mg. An equivalent amount of sitagliptin phosphate monohydrate to the sitagliptin free base anhydrate is used in the pharmaceutical compositions, namely, 32.13, 64.25, 96.38, 128.5, 192.75, and 257 mg, respectively. Adjusted dosages of 25 and 50 mg sitagliptin are used for patients with renal failure. Typical dosage strengths of the dual combination of sitagliptin / metformin are 50/500 mg and 50/1000 mg.

A dosage range of the DPP-4 inhibitor vildagliptin is usually between 10 and 150 mg daily, in particular between 25 and 150 mg, 25 and 100 mg or 25 and 50 mg or 50 and 100 mg daily. Particular examples of daily oral dosage are 25, 30, 35, 45, 50, 55, 60, 80, 100 or 150 mg. In a more particular aspect, the daily administration of vildagliptin may be between 25 and 150 mg or between 50 and 100 mg. In another more particular aspect, the daily administration of vildagliptin may be 50 or 100 mg. The application of the active ingredient may occur up to three times a day, preferably one or two times a day. Particular dosage strengths are 50 mg or 100 mg vildagliptin. Typical dosage strengths of the dual combination of vildagliptin / metformin are 50/850 mg and 50/1000 mg.

Alogliptin may be administered to a patient at a daily dose of between 5 mg/day and 250 mg/day, optionally between 10 mg and 200 mg, optionally between 10 mg and 150 mg, and optionally between 10 mg and 100 mg of alogliptin (in each instance based on the molecular weight of the free base form of alogliptin). Thus, specific dosage amounts that may be used include, but are not limited to 10 mg, 12.5 mg, 20 mg, 25 mg, 50 mg, 75 mg and 100 mg of alogliptin per day. Alogliptin may be administered in its free base form or as a pharmaceutically acceptable salt.

Saxagliptin may be administered to a patient at a daily dose of between 2.5 mg/day and 100 mg/day, optionally between 2.5 mg and 50 mg. Specific dosage amounts that may be used include, but are not limited to 2.5 mg, 5 mg, 10 mg, 15 mg, 20 mg, 30 mg , 40 mg, 50 mg and 100 mg of saxagliptin per day. Typical dosage strengths of the dual combination of saxagliptin / metformin are 2.5/500 mg and 2.5/1000 mg.

A special embodiment of the DPP-4 inhibitors of this invention refers to those orally administered DPP-4 inhibitors which are therapeutically efficacious at low dose levels, e.g. at dose levels < 100 mg or < 70 mg per patient per day, preferably < 50 mg, more preferably < 30 mg or < 20 mg, even more preferably from 1 mg to 10 mg (if required, divided into 1 to 4 single doses, particularly 1 or 2 single doses, which may be of the same size), particularly from 1 mg to 5 mg (more particularly 5 mg), per patient per day, preferentially, administered orally once-daily, more preferentially, at any time of day, administered with or without food. Thus, for example, the daily oral amount 5 mg BI 1356 can be given in a once daily dosing regimen (i.e. 5 mg BI 1356 once daily) or in a twice daily dosing regimen (i.e. 2.5 mg BI 1356 twice daily), at any time of day, with or without food.

A particularly preferred DPP-4 inhibitor to be emphasized within the meaning of this invention is 1-[(4-methyl-quinazolin-2-yl)methyl]-3-methyl-7-(2-butyn-1-yl)-8-(3-(R)-amino-piperidin-1-yl)-xanthine (also known as BI 1356). BI 1356 exhibits high potency, 24h duration of action, and a wide therapeutic window. In patients with type 2 diabetes receiving multiple oral doses of 1, 2.5, 5 or 10 mg of BI 1356 once daily for 12 days, BI 1356 shows favourable pharmacodynamic and pharmacokinetic profile (see e.g. Table 1 below) with rapid attainment of steady state (e.g. reaching steady state plasma levels (> 90% of the pre-dose plasma concentration on Day 13) between second and fifth day of treatment in all dose groups), little accumulation (e.g. with a mean accumulation ratio R_{A,AUC} ≤ 1.4 with doses above 1 mg) and preserving a long-lasting effect on DPP-4 inhibition (e.g. with almost complete (> 90%) DPP-4 inhibition at the 5 mg and 10 mg dose levels, i.e. 92.3 and 97.3% inhibition at steady state, respectively, and > 80% inhibition over a 24h interval after drug intake), as well as significant decrease in 2h postprandial blood glucose excursions by ≥ 80 % (already on Day 1) in doses ≥ 2.5 mg, and with the cumulative amount of unchanged parent compound excreted in urine on Day 1 being below 1% of the administered dose and increasing to not more than about 3-6% on Day 12 (renal clearance CL_{R,ss} is from about 14 to about 70 mL/min for the administered oral doses, e.g. for the 5 mg dose renal clearance is about 70 ml/min). In people with type 2 diabetes BI 1356 shows a placebo-like safety and tolerability. With low doses of about ≥ 5 mg, BI 1356 acts as a true once-daily oral drug with a full 24 h duration of DPP-4 inhibition. At therapeutic oral dose levels, BI 1356 is mainly excreted via the liver and only to a minor extent (about < 7% of the administered oral dose) via the kidney. BI 1356 is primarily excreted unchanged via the bile. The fraction of BI 1356 eliminated via the kidneys increases only very slightly over time and with increasing dose, so that there will likely be no need to modify the dose of BI 1356 based on the patients' renal function. The non-renal elimination of BI 1356 in combination with its low accumulation potential and broad safety margin may be of significant benefit in a patient population that has a high prevalence of renal insufficiency and diabetic nephropathy.

**Table 1: Geometric mean (gMean) and geometric coefficient of variation (gCV) of pharmacokinetic parameters of BI 1356 at steady state (Day 12)**

| **Parameter** | **1 mg gMean (gCV)** | **2.5 mg gMean (gCV)** | **5 mg gMean (gCV)** | **10 mg gMean (gCV)** |
|---|---|---|---|---|
| AUC₀₋₂₄ [nmol·h/L] | 40.2 (39.7) | 85.3 (22.7) | 118 (16.0) | 161 (15.7) |
| AUC_{τ,ss} [nmol·h/L] | 81.7 (28.3) | 117 (16.3) | 158 (10.1) | 190 (17.4) |
| Cₘₐₓ [nmol/L] | 3.13 (43.2) | 5.25 (24.5) | 8.32 (42.4) | 9.69 (29.8) |
| C_{max,ss} [nmol/L] | 4.53 (29.0) | 6.58 (23.0) | 11.1 (21.7) | 13.6 (29.6) |
| tₘₐₓ* [h] | 1.50 [1.00 - 3.00] | 2.00 [1.00 - 3.00] | 1.75 [0.92 - 6.02] | 2.00 [1.50 - 6.00] |
| t_{max,ss}* [h] | 1.48 [1.00 - 3.00] | 1.42 [1.00 - 3.00] | 1.53 [1.00 - 3.00] | 1.34 [0.50 - 3.00] |
| T_{½,ss} [h] | 121 (21.3) | 113 (10.2) | 131 (17.4) | 130 (11.7) |
| Accumulation t_{1/2,} [h] | 23.9 (44.0) | 12.5 (18.2) | 11.4 (37.4) | 8.59 (81.2) |
| R_{A,Cmax} | 1.44 (25.6) | 1.25 (10.6) | 1.33 (30.0) | 1.40 (47.7) |
| R_{A,AUC} | 2.03 (30.7) | 1.37 (8.2) | 1.33 (15.0) | 1.18 (23.4) |
| fe₀₋₂₄ [%] | NC | 0.139 (51.2) | 0.453 (125) | 0.919 (115) |
| fe_{τ,ss} [%] | 3.34 (38.3) | 3.06 (45.1) | 6.27 (42.2) | 3.22 (34.2) |
| CL_{R,ss} [mL/min] | 14.0 (24.2) | 23.1 (39.3) | 70 (35.0) | 59.5 (22.5) |

| | | | | |
|---|---|---|---|---|
| * median and range [min-max] NC not calculated as most values below lower limit of quantification | | | | |

As different metabolic functional disorders often occur simultaneously, it is quite often indicated to combine a number of different active principles with one another. Thus, depending on the functional disorders diagnosed, improved treatment outcomes may be obtained if a DPP-4 inhibitor is combined with active substances customary for the respective disorders, such as e.g. one or more active substances selected from among the other antidiabetic substances, especially active substances that lower the blood sugar level or the lipid level in the blood, raise the HDL level in the blood, lower blood pressure or are indicated in the treatment of atherosclerosis or obesity.

The DPP-4 inhibitors mentioned above - besides their use in mono-therapy - may also be used in conjunction with other active substances, by means of which improved treatment results can be obtained. Such a combined treatment may be given as a free combination of the substances or in the form of a fixed combination, for example in a tablet or capsule. Pharmaceutical formulations of the combination partner needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ^{®}" of the federal association of the pharmaceutical industry, or in the annually updated compilation of manufacturers' information on prescription drugs known as the "Physicians' Desk Reference".

Examples of antidiabetic combination partners are metformin; sulphonylureas such as glibenclamide, tolbutamide, glimepiride, glipizide, gliquidon, glibornuride and gliclazide; nateglinide; repaglinide; thiazolidinediones such as rosiglitazone and pioglitazone; PPAR gamma modulators such as metaglidases; PPAR-gamma agonists such as GI 262570; PPAR-gamma antagonists; PPAR-gamma/alpha modulators such as tesaglitazar, muraglitazar, aleglitazar, indeglitazar and KRP297; PPAR-gamma/alpha/delta modulators; AMPK-activators such as AICAR; acetyl-CoA carboxylase (ACC1 and ACC2) inhibitors; diacylglycerol-acetyltransferase (DGAT) inhibitors; pancreatic beta cell GCRP agonists such as SMT3-receptor-agonists and GPR119; 11β-HSD-inhibitors; FGF19 agonists or analogues; alpha-glucosidase blockers such as acarbose, voglibose and miglitol; alpha2-antagonists; insulin and insulin analogues such as human insulin, insulin lispro, insulin glusilin, r-DNA-insulinaspart, NPH insulin, insulin detemir, insulin zinc suspension and insulin glargin; Gastric inhibitory Peptide (GIP); pramlintide, davalintide; amylin and amylin analogues or GLP-1 and GLP-1 analogues such as Exendin-4, e.g. exenatide, exenatide LAR, liraglutide, taspoglutide, AVE-0010, LY-2428757, LY-2189265, semaglutide or albiglutide; SGLT2-inhibitors such as KGT-1251; inhibitors of protein tyrosine-phosphatase; inhibitors of glucose-6-phosphatase; fructose-1,6-bisphosphatase modulators; glycogen phosphorylase modulators; glucagon receptor antagonists; phosphoenolpyruvatecarboxykinase (PEPCK) inhibitors; pyruvate dehydrogenasekinase (PDK) inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); glucokinase/regulatory protein modulators incl. glucokinase activators; glycogen synthase kinase inhibitors; inhibitors of the SH2-domain-containing inositol 5-phosphatase type 2 (SHIP2) ; IKK inhibitors such as highdose salicylate ; JNK1 inhibitors ; protein kinase C-theta inhibitors; beta 3 agonists such as ritobegron, YM 178, solabegron, talibegron, N-5984, GRC-1087, rafabegron, FMP825; aldosereductase inhibitors such as AS 3201, zenarestat, fidarestat, epalrestat, ranirestat, NZ-314, CP-744809, and CT-112; SGLT-1 or SGLT-2 inhibitors, such as e.g. dapagliflozin, sergliflozin, atigliflozin, larnagliflozin or canagliflozin (or compound of formula (I-S) or (I-K) from WO 2009/035969); KV 1.3 channel inhibitors; GPR40 modulators; SCD-1 inhibitors; CCR-2 antagonists; dopamine receptor agonists (bromocriptine mesylate [Cycloset]); and other DPP IV inhibitors.

Metformin is usually given in doses varying from about 500 mg to 2000 mg up to 2500 mg per day using various dosing regimens from about 100 mg to 500 mg or 200 mg to 850 mg (1-3 times a day), or about 300 mg to 1000 mg once or twice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

A dosage of pioglitazone is usually of about 1-10 mg, 15 mg, 30 mg, or 45 mg once a day.

Rosiglitazone is usually given in doses from 4 to 8 mg once (or divided twice) a day (typical dosage strengths are 2, 4 and 8 mg).

Glibenclamide (glyburide) is usually given in doses from 2.5-5 to 20 mg once (or divided twice) a day (typical dosage strengths are 1.25, 2.5 and 5 mg), or micronized glibenclamide in doses from 0.75-3 to 12 mg once (or divided twice) a day (typical dosage strengths are 1.5, 3, 4.5 and 6 mg).

Glipizide is usually given in doses from 2.5 to 10-20 mg once (up to 40 mg divided twice) a day (typical dosage strengths are 5 and 10 mg), or extended-release glipizide in doses from 5 to 10 mg (up to 20 mg) once a day (typical dosage strengths are 2.5, 5 and 10 mg).

Glimepiride is usually given in doses from 1-2 to 4 mg (up to 8 mg) once a day (typical dosage strengths are 1, 2 and 4 mg).

A dual combination of glibenclamide/metformin is usually given in doses from 1.25/250 once daily to 10/1000 mg twice daily (typical dosage strengths are 1.25/250, 2.5/500 and 5/500 mg).

A dual combination of glipizide/metformin is usually given in doses from 2.5/250 to 10/1000 mg twice daily (typical dosage strengths are 2.5/250, 2.5/500 and 5/500 mg).

A dual combination of glimepiride/metformin is usually given in doses from 1/250 to 4/1000 mg twice daily.

A dual combination of rosiglitazone/glimepiride is usually given in doses from 4/1 once or twice daily to 4/2 mg twice daily (typical dosage strengths are 4/1, 4/2, 4/4, 8/2 and 8/4 mg).

A dual combination of pioglitazone/glimepiride is usually given in doses from 30/2 to 30/4 mg once daily (typical dosage strengths are 30/4 and 45/4 mg).

A dual combination of rosiglitazone/metformin is usually given in doses from 1/500 to 4/1000 mg twice daily (typical dosage strengths are 1/500, 2/500, 4/500, 2/1000 and 4/1000 mg).

A dual combination of pioglitazone/metformin is usually given in doses from 15/500 once or twice daily to 15/850 mg thrice daily (typical dosage strengths are 15/500 and 15/850 mg).

The non-sulphonylurea insulin secretagogue nateglinide is usually given in doses from 60 to 120 mg with meals (up to 360 mg/day, typical dosage strengths are 60 and 120 mg); repaglinide is usually given in doses from 0.5 to 4 mg with meals (up to 16 mg/day, typical dosage strengths are 0.5, 1 and 2 mg). A dual combination of repaglinide/metformin is available in dosage strengths of 1/500 and 2/850 mg.

Acarbose is usually given in doses from 25 to 100 mg with meals (up to 300 mg/day, typical dosage strengths are 25, 50 and 100 mg). Miglitol is usually given in doses from 25 to 100 mg with meals (up to 300 mg/day, typical dosage strengths are 25, 50 and 100 mg). Conventional antidiabetics and antihyperglycemics typically used in mono- or dual or triple (add-on or initial) combination therapy may include, without being limited to, metformin, sulphonylureas, thiazolidinediones, glinides, alpha-glucosidase blockers, GLP-1 and GLP-1 analogues, as well as insulin and insulin analogues, such as e.g. those agents indicated herein by way of example, including combinations thereof.

Examples of combination partners that lower the lipid level in the blood are HMG-CoA-reductase inhibitors such as simvastatin, atorvastatin, lovastatin, fluvastatin, pravastatin, pitavastatin and rosuvastatin; fibrates such as bezafibrate, fenofibrate, clofibrate, gemfibrozil, etofibrate and etofyllinclofibrate; nicotinic acid and the derivatives thereof such as acipimox; PPAR-alpha agonists; PPAR-delta agonists; inhibitors of acyl-coenzyme A:cholesterolacyltransferase (ACAT; EC 2.3.1.26) such as avasimibe; cholesterol resorption inhibitors such as ezetimib; substances that bind to bile acid, such as cholestyramine, colestipol and colesevelam; inhibitors of bile acid transport; HDL modulating active substances such as D4F, reverse D4F, LXR modulating active substances and FXR modulating active substances; CETP inhibitors such as torcetrapib, JTT-705 (dalcetrapib) or compound 12 from WO 2007/005572 (anacetrapib); LDL receptor modulators; and ApoB100 antisense RNA.

A dosage of atorvastatin is usually from 1 mg to 40 mg or 10 mg to 80 mg once a day.

Examples of combination partners that lower blood pressure are beta-blockers such as atenolol, bisoprolol, celiprolol, metoprolol and carvedilol; diuretics such as hydrochlorothiazide, chlortalidon, xipamide, furosemide, piretanide, torasemide, spironolactone, eplerenone, amiloride and triamterene; calcium channel blockers such as amlodipine, nifedipine, nitrendipine, nisoldipine, nicardipine, felodipine, lacidipine, lercanipidine, manidipine, isradipine, nilvadipine, verapamil, gallopamil and diltiazem; ACE inhibitors such as ramipril, lisinopril, cilazapril, quinapril, captopril, enalapril, benazepril, perindopril, fosinopril and trandolapril; as well as angiotensin II receptor blockers (ARBs) such as telmisartan, candesartan, valsartan, losartan, irbesartan, olmesartan and eprosartan.

A dosage of telmisartan is usually from 20 mg to 320 mg or 40 mg to 160 mg per day.

Examples of combination partners which increase the HDL level in the blood are Cholesteryl Ester Transfer Protein (CETP) inhibitors; inhibitors of endothelial lipase; regulators of ABC1; LXRalpha antagonists; LXRbeta agonists; PPAR-delta agonists; LXRalpha/beta regulators, and substances that increase the expression and/or plasma concentration of apolipoprotein A-I.

Examples of combination partners for the treatment of obesity are sibutramine; tetrahydrolipstatin (orlistat); alizyme; dexfenfluramine; axokine; cannabinoid receptor 1 antagonists such as the CB1 antagonist rimonobant; MCH-1 receptor antagonists; MC4 receptor agonists; NPY5 as well as NPY2 antagonists; beta3-AR agonists such as SB-418790 and AD-9677; 5HT2c receptor agonists such as APD 356 (lorcaserin); myostatin inhibitors; Acrp30 and adiponectin; steroyl CoA desaturase (SCD1) inhibitors; fatty acid synthase (FAS) inhibitors; CCK receptor agonists; Ghrelin receptor modulators; Pyy 3-36; orexin receptor antagonists; and tesofensine; ; as well as the dual combinations bupropion/naltrexone, bupropion/zonisamide, topiramate/phentermine and pramlintide/metreleptin.

Examples of combination partners for the treatment of atherosclerosis are phospholipase A2 inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); oxLDL antibodies and oxLDL vaccines; apoA-1 Milano; ASA; and VCAM-1 inhibitors.

Further embodiments, features and advantages of the present invention may become apparent from the following examples. The following examples serve to illustrate, by way of example, the principles of the invention without restricting it.

### Examples

### Animal model:

Sulfonylurea (SU) like glibenclamide are one of the most frequently used drugs in diabetes treatment. Long-term treatment with SU causes elevated basal insulin secretion and decreased glucose-stimulated insulin secretion. These characteristics may play an important role for the development of hypoglycemia and secondary drug failure. Db/db mice represent an animal model for type 2 diabetes demonstrating insulin resistance and high levels of plasma glucose. In addition, correlating with age of the animals pancreatic β-cells of aging db/db mice fails to compensate the high glucose excursion with enhanced insulin secretion. Therefore this model is appropriate to study the glibenclamide induced secondary drug failure in comparison to a DPP-4 inhibitor (e.g. BI 1356).

### Methods

### Animals and housing

Female db/db mice at 5 weeks of age, are obtained from Charles River, Germany. Animals are housed in groups of 5-6 animals under a 12:12 L/D cycle (lights on at 04:00 AM and lights off at 04:00 PM) in temperature and humidity controlled rooms. All animals have free access to regular rodent chow (Altromin standard #1324 chow, Denmark) and water ad libitum.

### In Vivo experiment

The compound solutions are administered daily at 08.00 AM on experimental days 0-59 by per oral gavage using a gastric tube connected to a 3 ml syringe (luer-lock^{™}, Becton). Groups of 12 animals are used: vehicle, 0.5% Natrosol; BI 1356 3 mg/kg; glibenglamid 3 mg/kg. Body weight food intake and water intake is recorded daily for the first two and twice weekly for the remaining period. On experimental day 54 blood glucose and HbA1c levels are determined in semi-fed state, on day 59 an OGTT (2 g/kg) is performed.

### HbA1c, insulin and blood glucose monitoring

Blood samples for determination of "fed" levels blood glucose and HbA1c are performed at 10.00 AM on day 54. Prior to blood sampling animals are transferred to clean cages with no food two hours before blood sampling. At day 59 an OGTT (2g/kg) is performed following an over-night fast of the animals and insulin is detected at t=15 min.

**Blood glucose:** For each data point, 10µl blood is drawn from the tip of the tail into a microcapillary tube and measured using a Biosen S line glucose analyzer.

**Insulin:** For each data point, 100 µl blood is drawn from the tail vein, collected in EDTA tubes. Insulin is measured using a Mouse Endocrine Immunoassay Panel (LINCOplex^{™}, analyzed using a Luminex100TM system; LincoResearch, Missouri, USA).

**HbA1c:** are measured using a standard enzyme assay kit on a fully automated analyzer (Bayer).

### Results

Fig 1. and 2 demonstrate HbA1c and glucose values following 54 day treatment of 5 weeks old female db/db mice with the indicated compounds. The DPP-4 inhibitor BI 1356 improves HbA1c as well as glucose values compared to control. In contrast, the sulfonylurea glibenclamide in a concentration of 3 mg/kg impairs glucose as well as HbA1c values compared to control and BI 1356.

Fig. 3 shows the increase in insulin during an OGTT test. Only the animals treated with BI 1356 are able to respond to the increased glucose levels with an up-regulation of insulin.

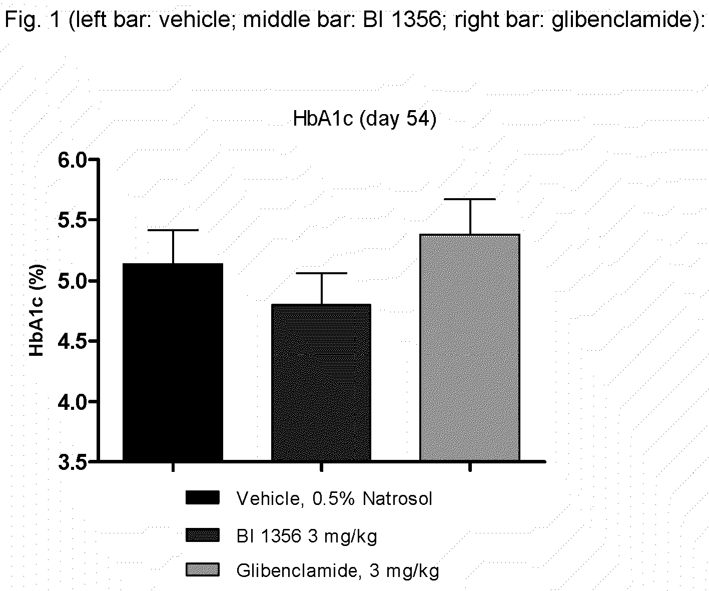

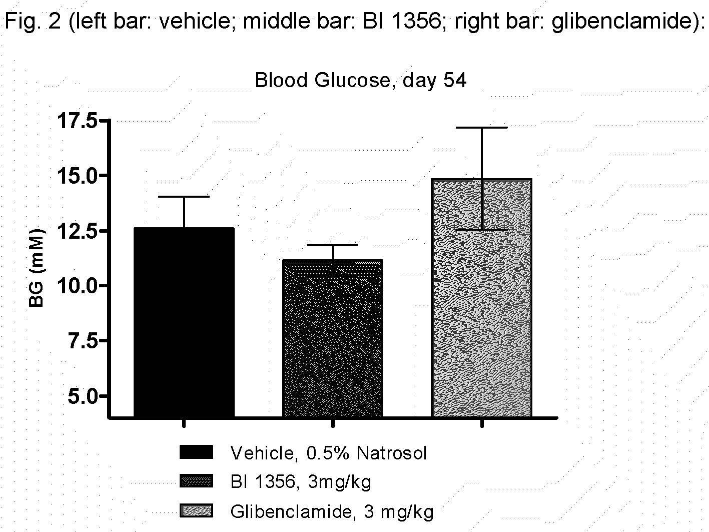

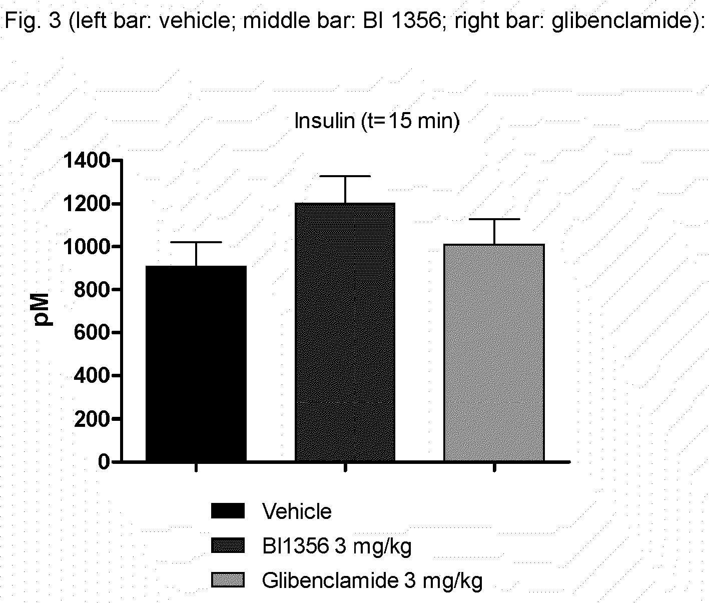

Thus, in an animal model representing β-cell and SU induced secondary drug failure the DPP-4 inhibitor BI 1356 is superior to glibenclamide regarding insulin secretion and lowering of HbA1c and glucose.

### Clinic:

The usability of a DPP-4 inhibitor according to this invention for the purpose of the present invention can be tested using clinical trials:
For example, in a randomised, double-blind, placebo-controlled, parallel group trial, the safety and efficacy of a DPP-4 inhibitor according to the invention (e.g. 5 mg of BI 1356 administered orally once daily) is tested in patients with type 2 diabetes with insufficient glycemic control (HbA1c from 7.0 % to 10% or from 7.5 % to 10% or 11%) despite a therapy with one or two conventional antihyperglycemic agents, such as e.g. a sulphonylurea drug.

In the study with the sulphonylurea drug the efficacy and safety of a DPP-4 inhibitor according to this invention versus placebo added to a background therapy of a sulphonylurea is investigated (2 week placebo run-in phase; 18 weeks double-blind treatment followed by 1 week follow up after study medication termination; background therapy with a sulphonylurea drug is administered throughout the entire trial duration, including placebo run-in phase, in an unchanged dosage).

The success of the treatment is tested by determining the HbA1c value, by comparison with the initial value and/or with the value of the placebo group. A significant change in the HbA1c value compared with the initial value and/or the placebo value demonstrates the efficacy of the DPP-4 inhibitor for the treatment. The success of the treatment can be also tested by determining the fasting plasma glucose values, by comparison with the initial values and/or with the values of the placebo group. A significant drop in the fasting glucose levels demonstrates the efficacy of the treatment. Also, the occurrence of a treat to target response (i.e. an HbA1c under treatment < 7%) demonstrates the efficacy of the treatment.

The safety and tolerability of the treatment is investigated by assessing patient's condition and relevant changes from baseline, e.g. incidence and intensity of adverse events (such as e.g. hypoglycaemic episodes or the like) or weight gain.

## Claims

1. A DPP-4 inhibitor, which is 1-[(4-methyl-quinazolin-2-yl)methyl]-3-methyl-7-(2-butyn-1-yl)-8-(3-(R)-amino-piperidin-1-yl)-xanthine, or a pharmaceutically acceptable salt thereof, for use in a method for improving HbA1c for treating and/or preventing of a metabolic disease, selected from diabetes, especially type 2 diabetes mellitus, and diabetic complications, in patients with insufficient glycemic control despite a therapy with a sulphonylurea drug alone.

2. The DPP-4 inhibitor for use according to claim 1, wherein said DPP-4 inhibitor is used in combination with said sulphonylurea drug.

3. The DPP-4 inhibitor for use according to claim 1, wherein said DPP-4 inhibitor is used as replacement of said sulphonylurea.

4. The DPP-4 inhibitor for use according to any one of claims 1 to 3, wherein said sulphonylurea is selected from glibenclamide, glipizide and glimepiride.

5. The DPP-4 inhibitor for use according to any one of claims 1 to 4, wherein FPG and/or PPG is improved, glucose excursion is decreased and/or insulin secretion is improved in said patients.

6. The DPP-4 inhibitor for use according to any one of claims 1 to 5, wherein said patients have inadequate HbA1c values from 7.5 to 11% or 7.0 to 10% or 7.5 to 10% despite therapy with a sulphonylurea drug.

7. The DPP-4 inhibitor for use according to any one of the preceding claims, wherein the DPP-4 inhibitor is administered in a daily oral amount of 5 mg.

8. The DPP-4 inhibitor for use according to any one of claims 1 to 3, wherein said DPP-4 inhibitor or pharmaceutically acceptable salt thereof is for use in a method for improving HbA1c for treating type 2 diabetes mellitus in patients with insufficient glycemic control despite a therapy with a sulphonylurea drug alone.

## Patentansprüche

1. DPP-4-Inhibitor, der 1-[(4-methylchinazolin-2-yl)methyl]-3-methyl-7-(2-butin-1-yl)-8-(3-(R)-aminopiperidin-1-yl)-xanthin oder ein pharmazeutisch annehmbares Salz davon ist, zur Verwendung in einem Verfahren zur Verbesserung von HbA1c zur Behandlung und/oder Verhinderung einer Stoffwechselerkrankung, ausgewählt aus Diabetes, insbesondere Diabetes mellitus Typ 2, und diabetischen Komplikationen bei Patienten mit unzureichender glykämischer Kontrolle trotz einer Therapie mit einem Sulfonylharnstoffmedikament allein.

2. DPP-4-Inhibitor zur Verwendung nach Anspruch 1, wobei der DPP-4-Inhibitor in Kombination mit dem Sulfonylharnstoffmedikament verwendet wird.

3. DPP-4-Inhibitor zur Verwendung nach Anspruch 1, wobei der DPP-4-Inhibitor als Ersatz für das Sulfonylharnstoffmedikament verwendet wird.

4. DPP-4-Inhibitor zur Verwendung nach einem der Ansprüche 1 bis 3, wobei das Sulfonylharnstoffmedikament ausgewählt ist aus Glibenclamid, Glipizid und Glimepirid.

5. DPP-4-Inhibitor zur Verwendung nach einem der Ansprüche 1 bis 4, wobei bei den Patienten FPG und/oder PPG verbessert wird, die Glukoseexkursion verringert wird und/oder die Insulinsekretion verbessert wird.

6. DPP-4-Inhibitor zur Verwendung nach einem der Ansprüche 1 bis 5, wobei die Patienten trotz Therapie mit einem Sulfonylharnstoffmedikament unzureichende HbA1c-Werte von 7,5 bis 11 % oder 7,0 bis 10 % oder 7,5 bis 10 % aufweisen.

7. DPP-4-Inhibitor zur Verwendung nach einem der vorstehenden Ansprüche, wobei der DPP-4-Inhibitor in einer täglichen oralen Menge von 5 mg verabreicht wird.

8. DPP-4-Inhibitor zur Verwendung nach einem der Ansprüche 1 bis 3, wobei der DPP-4-Inhibitor oder das pharmazeutisch annehmbare Salz davon zur Verwendung in einem Verfahren zur Verbesserung von HbA1c zur Behandlung von Diabetes mellitus Typ 2 bei Patienten mit unzureichender glykämischer Kontrolle trotz einer Therapie mit einem Sulfonylharnstoffmedikament allein ist.

## Revendications

1. Inhibiteur de la DPP-4, qui est 1-[(4-méthyl-quinazolin-2-yl)méthyl]-3-méthyl-7-(2-butyn-1-yl)-8-(3-(R)-amino-pipéridin-1-yl)-xanthine, ou un sel pharmaceutiquement acceptable de celui-ci, destiné à être utilisé dans un procédé d'amélioration de l'HbA1c en vue du traitement et/ou de la prévention d'une maladie métabolique, choisie parmi le diabète, en particulier le diabète sucré de type 2, et les complications diabétiques, chez des patients dont la régulation glycémique est insuffisante malgré une thérapie par une sulfonylurée seule.

2. Inhibiteur de la DPP-4 destiné à être utilisé selon la revendication 1, dans lequel ledit inhibiteur de la DPP-4 est utilisé en combinaison avec ladite sulfonylurée.

3. Inhibiteur de la DPP-4 destiné à être utilisé selon la revendication 1, dans lequel ledit inhibiteur de la DPP-4 est utilisé en remplacement de ladite sulfonylurée.

4. Inhibiteur de la DPP-4 destiné à être utilisé selon l'une quelconque des revendications 1 à 3, dans lequel ladite sulfonylurée est choisie parmi le glibenclamide, le glipizide et le glimépiride.

5. Inhibiteur de la DPP-4 destiné à être utilisé selon l'une quelconque des revendications 1 à 4, dans lequel le FPG et/ou le PPG est amélioré, l'excursion glycémique est diminuée et/ou la sécrétion d'insuline est améliorée chez lesdits patients.

6. Inhibiteur de la DPP-4 destiné à être utilisé selon l'une quelconque des revendications 1 à 5, dans lequel lesdits patients présentent des valeurs d'HbA1c inadéquates de 7,5 % à 11 % ou de 7,0 % à 10 % ou de 7,5 % à 10 % malgré une thérapie par une sulfonylurée.

7. Inhibiteur de la DPP-4 destiné à être utilisé selon l'une quelconque des revendications précédentes, dans lequel l'inhibiteur de la DPP-4 est administré à une dose orale quotidienne de 5 mg.

8. Inhibiteur de la DPP-4 destiné à être utilisé selon l'une quelconque des revendications 1 à 3, dans lequel ledit inhibiteur de la DPP-4 ou un sel pharmaceutiquement acceptable de celui-ci est destiné à être utilisé dans un procédé d'amélioration de l'HbA1c en vue du traitement du diabète sucré de type 2 chez des patients dont la régulation glycémique est insuffisante malgré une thérapie par une sulfonylurée seule.
